# EUROPÄISCHE PATENTSCHRIFT

(11) **EP 1 127 052 B1**
(45) Veröffentlichungstag und Bekanntmachung des Hinweises auf die Patenterteilung: **08.12.2004**
(21) Anmeldenummer: 99955894.3
(22) Anmeldetag: 28.10.1999
(51) Int. Cl.: C07D 235/18, C07D 403/10, A61K 31/4184, C07C 237/30, G01N 33/573

(54) **SUBSTITUIERTE 2-PHENYLBENZIMIDAZOLE, DEREN HERSTELLUNG UND ANWENDUNG**
SUBSTITUTED 2-PHENYLBENZIMIDAZOLES, THE PRODUCTION THEREOF AND THEIR USE
2-PHENYLBENZIMIDAZOLES SUBSTITUES, LEUR PREPARATION ET LEUR UTILISATION

(30) Priorität: 03.11.1998 DE 19850709; 16.11.1998 DE 19852801; 01.03.1999 DE 19908733
(43) Veröffentlichungstag der Anmeldung: 29.08.2001
(60) Teilanmeldung: 03024899.1
(73) Patentinhaber: Abbott GmbH & Co. KG, 65205 Wiesbaden (DE)
(72) Erfinder: LUBISCH, Wilfried, D-69115 Heidelberg (DE); KOCK, Michael, D-67105 Schifferstadt (DE); HÖGER, Thomas, D-68535 Edingen-Neckarhausen (DE)
(74) Vertreter: Grünecker, Kinkeldey, Stockmair & Schwanhäusser Anwaltssozietät
(86) Internationale Anmeldenummer: PCT/EP1999/008169
(87) Internationale Veröffentlichungsnummer: WO 2000/026192

(56) Entgegenhaltungen:
- EP-A- 0 209 707
- EP-A- 0 719 765
- WO-A-97/04771
- DE-A- 2 248 596
- FR-A- 2 707 011
- GILCHRIST T L: "CYCLISATION OF ORTHO-SUBSTITUTED N-ARYLBENZIMIDOYL NITRENES. PART 2.1 PREFERENTIAL CYCLISATIONS AT AN ORTHO-POSITION BEARING A METHOXYCARBONYL GROUP" JOURNAL OF THE CHEMICAL SOCIETY, PERKIN TRANSACTIONS 1,GB,CHEMICAL SOCIETY. LETCHWORTH,1. Januar 1979 (1979-01-01), Seiten 2303-2307, XP000605168 ISSN: 0300-922X in der Anmeldung erwähnt
- CHEMICAL ABSTRACTS, vol. 125, no. 15, 7. Oktober 1996 (1996-10-07) Columbus, Ohio, US; abstract no. 189126j, ROGER J. GRIFFIN ET AL: "Resistance modifying agents.3. Novel benzimidazole and quinnazolinone inhibitors of the DNA repair enzyme poly(ADP-ribose)polymerase" Seite 521; XP002128895 & PHARM. SCI., Bd. 2, Nr. 1, 1996, Seiten 43-47,
- CHEMICAL ABSTRACTS, vol. 74, no. 9, 1. März 1971 (1971-03-01) Columbus, Ohio, US; abstract no. 42311k, YOSHUKE OHKURA ET AL: "Organic analysis. LXXII. Mechanism of the color reaction between m-dinitrobenzene and alkali cyanide. II.Color reactions products of 2,4-dinitroaniline with potassium cyanide" Seite 347; XP002128896 & CHEM. PHARM. BULL., Bd. 18, Nr. 11, 1970, Seiten 2164-2168,

## Beschreibung

Die vorliegende Erfindung betrifft neuartige 2-Phenylbenzimidazole, ihre Herstellung mit neuen Zwischenprodukten und die Verwendung als Inhibitoren des Enzyms Poly(ADP-ribose)polymerase oder PARP (EC 2.4.2.30) zur Herstellung von Arzneimitteln.

Poly(ADP-ribose)polymerase (PARP), bzw. wie es auch genannt wird Poly(ADP-ribose)synthase (PARS), stellt ein regulatorisches Enzym dar, das in Zellkernen gefunden wird (K. Ikai et al., *J. Histochem. Cytochem*. 1983, *31,* 1261-1264). Man nimmt an, daß PARP eine Rolle bei der Reparatur von DNA-Brüchen spielt (M.S. Satoh et al., *Nature* 1992, *356,* 356-358). Schädigungen oder Brüche der DNA-Stränge aktivieren das Enzym PARP, das, wenn es aktiviert ist, die Übertragung von ADP-Ribose aus NAD katalysiert (S. Shaw, *Adv. Radiat. Biol*., 1984, *11,* 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei einer Reihe von pathophysiologischen Zuständen beobachtet und man geht davon aus, daß diese Anhäufung von Radikalen zu den beobachteten Zell- bzw. Organschäden führen oder beitragen. Dazu zählt von zum Beispiel ischämische Zustände von Organen wie im Schlaganfall, Herzinfarkt (C. Thiemermann et al., *Proc. Natl. Acad. Sci. USA*, 1997, *94,* 679-683) oder Ischämie der Nieren, aber auch Reperfusionsschäden wie sie zum Beispiel nach der Lyse von Herzinfarkt auftreten (s. oben: C. Thiemermann et al.). Die Hemmung von dem Enzym PARP könnte demzufolge ein Mittel sein, um diese Schäden mindestens zum Teil zu verhindern oder abzumildern. PARP-Inhibitoren könnten somit ein neues Therapieprinzip zur Behandlung von einer Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. *Cancer Chemo. Pharmacöl*. 1988, *22,* 303).

Nicht limitierende Beispiele für Tumoren sind Leukämie, Glioblastome, Lymphome, Melanome, Mama- und Cervicalkarzinome.

Zudem wurde gefunden, daß PARP-Inhibitoren immunosuppressive Wirkung zeigen können (D. Weltin et al. *Int. J. Immunopharmacol*. 1995, *17,* 265-271).

Es wurde ebenfalls entdeckt, daß PARP bei immunologischen Erkrankungen bzw. Krankheiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. *Infammation* 1996, *20,* 203-215; W. Ehrlich et al. *Rheumatol. Int.* 1995, *15*, 171-172; C. Szabo et al., *Proc*. *Natl*. *Acad. Sci. USA* 1998, *95,* 3867-3872; S. Cuzzocrea et al. *Eur. J. Pharmacol*. 1998, *342,* 67-76).

Unter PARP im Sinne dieser Erfindung werden auch Isoenzyme des oben beschriebenen PARP-Enzyms verstanden. Solche Isoenzyme sind z.B. PARP II und PARP III.

Weiterhin zeigte der PARP-Inhibitor 3-Aminobenzamid protektive Effekte in einem Model für den Kreislaufschock (S. Cuzzocrea et al., *Br. J. Pharmacol.* 1997, *121,* 1065-1074).

2-Phenylbenzimidazole sind vielfach beschrieben worden. So sind in DE 38 30 060 alkylierte Derivate als Inhibitoren der Erythrozytenaggregation offengelegt. In DE 35 22 230 ist ein Ester-Derivat vom 2-Phenylbenzimidazol als Inhibitor der Plättchenaggragation aufgeführt. Halogen-substituierte 2-Phenylbenzimizaole, die am Phenyl-Ring substituierte Amin-Reste tragen, sind in WO 98/06703 als MCP-1-Antagonisten beschrieben worden.

Ebenfalls sind 2-Phenyl-benzimidazole bekannt, bei denen die Benzimidazol-Gruppe durch eine Amid-Gruppe substituiert ist. 5-Amido-Derivate des 2-Phenylbenzimidazols, die am Phenyl-Ring Alkyloxy-Reste tragen, sind in WO 94/12461 als Inhibitoren der cAMP-Phosphodiesterase beschrieben worden. Für analoge Derivate wurde in DE 35 46 575 (z.B. Beispiel 15) gefunden, daß diese Verbindungen positiv inotrope Effekte auslösen. Ebenfalls 4-Amido-Derivate, die in 3-Stellung einen Pyridyl-Rest tragen, sind in WO 97/48697 als Inhibitoren der cAMP-Phosphodiesterase aufgeführt.

Die Synthese von 2-Phenyl-benzimidazyl-4-amiden ist in J. Chem. Soc. Perkin Trans 1, 1979, 2303-2307, beschrieben worden. Analoge Verbindungen, die am Amid-Rest noch eine substituierte Alkyl-Kette tragen, und die cytotoxische Wirkung haben sollen, sind in J. Med. Chem. 1990, 33, 814-819, aufgeführt. In WO 97/04771 sind dagegen Benzimidazol-4-amide aufgeführt, die das PARS hemmen. Insbesondere sind Derivate dort als wirksam beschrieben, die einen Phenyl-Ring in 2-Stellung tragen, wobei der Phenyl-Ring noch mit einfachen Substituenten, wie Nitro, Methoxy und CF₃, substituiert sein kann. Obwohl diese Substanzen zum Teil gute Hemmung des Enzyms PARP zeigen, haben die dort beschriebenen Derivate den Nachteil, daß sie nur wenig oder keine Löslichkeit in wäßrigen Lösungen zeigen und somit nicht als wäßrige Lösung appliziert werden können.

In einer Reihe von Therapien wie Schlaganfall werden die Wirkstoffe intravenös als Infusionslösung appliziert. Dazu ist es notwendig, Substanzen, hier PARP-Inhibitoren, zur Verfügung zu haben, die ausreichende Wasserlöslichkeit bei physiologischen pH-Werten oder angenäherten pH-Werten (z.B: pH-Werten von 5 bis 8) aufweisen, so daß eine Infusionslösung hergestellt werden kann. Viele der beschriebenen PARP-Inhibitoren, insbesondere die besser wirksamen PARP-Inhibitoren, haben jedoch den Nachteil, daß sie nur geringe oder keine Wasserlöslichkeit bei diesen pH-Werten zeigen und somit nicht für eine intravenöse Applikation in Frage kommen. Derartige Wirkstoffe können nur mit Hilfsstoffen, die die Wasserlöslichkeit vermitteln sollen, appliziert werden (vgl. WO 97/04771). Diese Hilfsstoffe, zum Beispiel Polyethylenglykol und Dimethysulfoxid, verursachen häufig Nebeneffekte oder sind sogar unverträglich. Gut wirksame PARP-Inhibitoren mit ausreichender Wasserlöslichkeit sind bisher nicht beschrieben worden.

Es wurde überraschenderweise gefunden, daß 2-Phenyl-benzimidazole, die am Phenyl-Ring mit Alkoxy-Resten substituiert sind und an der Alkoxy-Seitenkette noch einen Amin-Rest tragen, gut wirksame Inhibitoren darstellen, die aber durch den Einbau des aliphatischen Amin-Restes eine Salzbildung mit Säuren ermöglichen und dadurch eine deutlich verbesserte Wasserlöslichkeit zeigen.

In der vorliegenden Erfindung werden neue 2-Phenylbenzimidazol-Derivate der allgemeinen Formel I beschrieben, die gegenüber den bereits beschriebenen Verbindungen Vorteile zeigen und potente PARP-Inhibitoren darstellen und zugleich auch ausreichende Wasserlöslichkeit zeigen, die eine Applikation als Infusionslösung ermöglicht.

Gegenstand der vorliegenden Erfindung sind substituierte 2-Phenylbenzimidazole der allgemeinen Formel I oder II 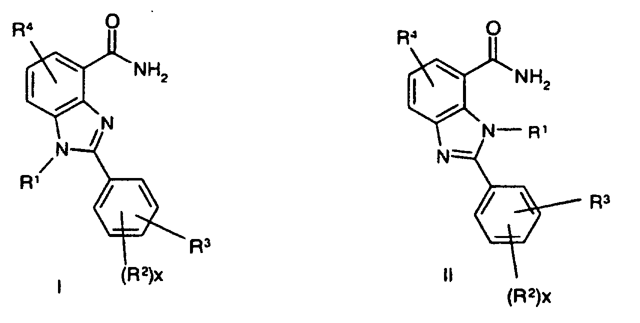 worin
- R¹: Wasserstoff, verzweigtes und unverzweigtes C₁-C₆-Alkyl, wobei ein C-Atom des Alkyl-Restes noch OR¹¹ oder eine Gruppe R⁵ tragen kann, wobei
R¹¹ Wasserstoff oder C₁-C₄-Alkyl bedeutet, und
- R²: Wasserstoff, Chlor, Brom, Jod, Fluor, CF₃, Nitro, NHCOR²¹, NR²²R²³ OH, O-C₁-C₄-Alkyl, O-C₁-C₄-Alkyl-Phenyl, NH₂, Phenyl, wobei die Phenyl-Ringe noch mit maximal zwei Resten R²⁴ substituiert sein können, und R²¹ und R²² unabhängig voneinander Wasserstoff oder C₁-C₄-Alkyl bedeuten und R²³ Wasserstoff, C₁-C₄-Alkyl oder Phenyl bedeuten, und R²⁴ OH, C₁-C₆-Alkyl, O-C₁-C₄-Alkyl, Chlor, Brom, Jod, Fluor, CF₃, Nitro, NH₂, und
- x: 0, 1 und 2 sein kann und
- R³: -D-(F¹)ₚ-(E)_{q}-(F²)ᵣ -G bedeutet, wobei p, q und r nicht gleichzeitig 0 sein können, oder -E-(D)ᵤ-(F²)ₛ-(G)ᵥ, wobei der Rest E noch mit einem oder zwei Resten A substituiert sein kann, der R³ g und
- R⁴: Wasserstoff, Chlor, Fluor, Brom, Jod, verzweigtes und unverzweigtes C₁-C₆-Alkyl, OH, Nitro, CF₃, CN, NR⁴¹R⁴², NH-CO-R⁴³, O-C₁-C₄-Alkyl, wobei
R⁴¹ und R⁴² unabhängig voneinander Wasserstoff oder C₁-C₄-Alkyl bedeuten und
R⁴³ Wasserstoff, C₁-C₄-Alkyl, C₁-C₄-Alkyl-Phenyl oder Phenyl bedeuten, und
- D: S und O
- E: Phenyl, Imidazol, Pyrrol, Thiophen, Pyridin, Pyrimidin, Piperazin, Pyrazin, Furan, Thiazol, Isoxazol, Pyrrolidin, Piperidin, Trihydroazepin und
- F¹: eine Kette aus 1 bis 8 Kohlenstoffatomen, wobei ein Kohlenstoffatom der Kette noch eine OH oder O-C₁-C₄-Alkyl-Gruppe tragen kann und
- F²: eine Kette aus 1 bis 8 Kohlenstoffatomen, wobei ein Kohlenstoffatom der Kette noch eine OH oder O-C₁-C₄-Alkyl-Gruppe tragen kann und
- p: 0 und 1 bedeuten kann und
- q: 0, und 1 sein kann, und
- r: 0 und 1 sein kann und
- s: 0 und 1 sein kann und
- u: 0 und 1 sein kann und
- v: 0 und 1 sein kann
G NR⁵¹R⁵² und 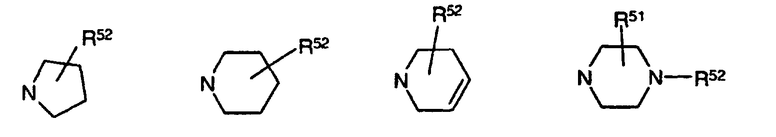 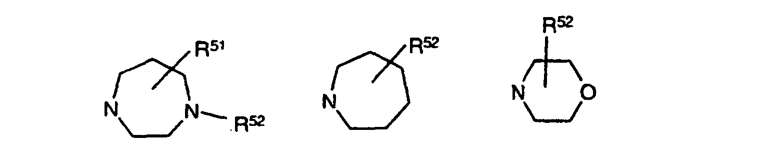 sein kann und
R⁵¹ Wasserstoff und verzweigtes und unverzweigtes C₁-C₆-Alkyl, (CH₂)ₜ-K bedeutet und
R⁵² Wasserstoff, verzweigtes und unverzweigtes C₁-C₆-Alkyl, Phenyl, 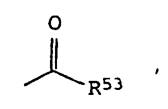 -SO₂R⁵³, -(C=N)-R⁵³, -CO-NHR⁵³, -(C=N)-NHR⁵³, worin
R⁵³ verzweigtes oder unverzweigtes O-C₁-C₆-Alkyl, Phenyl, verzweigtes oder unverzweigtes C₁-C₄-Alkyl-Phenyl,
wobei bei R⁵² und R⁵³ unabhängig voneinander ein Wasserstoff des C₁-C₆-Alkylrests durch einen der folgenden Reste substituiert sein kann: OH, O-C₁-C₄-Alkyl, Cyclohexyl, Cyclopentyl, Tetrahydronaphthyl, Cyclopropyl, Cyclobutyl, Cycloheptyl, Naphthyl und Phenyl, wobei die Carbocyclen der Reste R⁵² und R⁵³ unabhängig voneinander noch einen oder zwei der folgenden Reste tragen können: verzweigtes oder unverzweigtes C₁-C₆-Alkyl, verzweigtes oder unverzweigtes O-C₁-C₄-Alkyl, OH, F, Cl, Br, J, CF₃, NO₂, NH₂, CN, COOH, COOC₁-C₄-Alkyl, C₁-C₄-Alkylamino, CCl₃, C₁-C₄-Dialkylamino, SO₂-C₁-C₄-Alkyl, SO₂Phenyl, CONH₂, CONH-C₁-C₄-Alkyl, CONHPhenyl, CONH-C₁-C₄-Alkyl-Phenyl, NHSO₂-C₁-C₄-Alkyl, NHSO₂Phenyl, S-C₁-C₄-Alkyl, 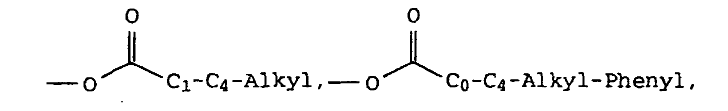 CHO, CH₂-O-C₁-C₄-Alkyl, -CH₂O-C₁-C₄-Alkyl-Phenyl, -CH₂OH, -SO-C₁-C₄-Alkyl, -SO-C₁-C₄-Alkyl-Phenyl, -SO₂NH₂, -SO₂NH-C₁-C₄-Alkyl
und zwei Reste eine Brücke -O-(CH₂)_{1,2}-O- bilden, bedeuten kann,
und
- A: Wasserstoff, Chlor, Brom, Jod, Fluor, CF₃, Nitro, OH, O-C₁-C₄-Alkyl, O-C₁-C₄-Alkyl-Phenyl, NH₂, verzweigtes und unverzweigtes C₁-C₆-Alkyl, CN, NH-CO-R³³, wobei R³³ Wasserstoff, C₁-C₄-Alkyl oder Phenyl bedeutet, sein kann und
- t: 0,1,2,3,4 und
- K: Phenyl, der noch maximal zwei Reste R tragen kann, NR^{k1}R^{k2} (mit R^{k1} bzw. R^{k2} mit den gleiche Bedeutungen wie R⁴¹ bzw. R⁴²), NH-C₁-C₄-Alkyl-Phenyl, Pyrrolidin, Piperidin, 1,2,5,6-Tetrahydropyridin, Morpholin, Trihydroazepin, Piperazin, das noch mit einem Alkyl-Rest C₁-C₆-Alkyl substituiert sein kann, und Homopiperazin, das noch mit einem Alkyl-Rest C₁-C₆-Alkyl substituiert sein kann, und
- R⁵: Wasserstoff, C₁-C₆-Alkyl, NR⁷R⁹ und 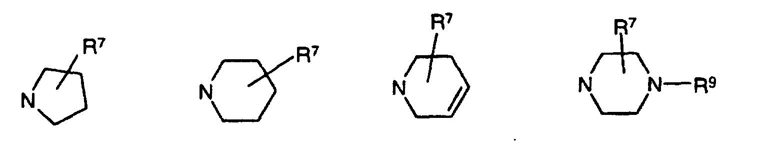 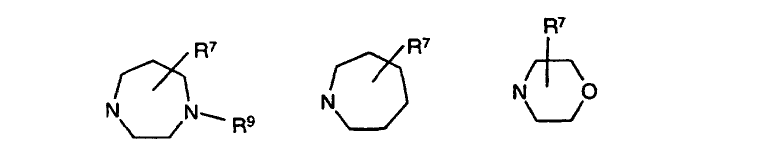 bedeuten kann und
- R⁷: Wasserstoff, C₁-C₆-Alkyl, C₁-C₄-Alkyl-Phenyl, Phenyl, wobei der Ring noch mit bis zu zwei Resten R⁷¹ substituiert sein können, und
- R⁷¹: OH, C₁-C₆-Alkyl, O-C₁-C₄-Alkyl, Chlor, Brom, Jod, Fluor, CF₃, Nitro, NH₂, und
- R⁸: Wasserstoff, C₁-C₆-Alkyl, Phenyl, C₁-C₄-Alkyl-Phenyl, wobei der Ring noch mit bis zu zwei Resten R⁸¹ substituiert sein kann, und
- R⁸¹: OH, C₁-C₆-Alkyl, O-C₁-C₄-Alkyl, Chlor, Brom, Jod, Fluor, CF₃, Nitro, NH₂, und
- R⁹: Wasserstoff, COCH₃, CO-O-C₁-C₄-Alkyl, COCF₃, verzweigtes und unverzweigtes C₁-C₆-Alkyl, wobei ein oder zwei Wasserstoffe des C₁-C₆-Alkylrests durch jeweils einen der folgenden Reste substituiert sein kann: OH, O-C₁-C₄-Alkyl und Phenyl und der Phenyl-Ring noch einen oder zwei der folgenden Reste tragen kann: Jod, Chlor, Brom, Fluor, verzweigtes und unverzweigtes C₁-C₆-Alkyl, Nitro, Amino, C₁-C₄-Alkylamino, C₁-C₄-Dialkylamino, OH, O-C₁-C₄-Alkyl, CN, CF₃, SO₂-C₁-C₄-Alkyl, bedeuten kann, und
sowie ihre tautomeren Formen, möglichen enantiomeren und diastereomeren Formen, und deren Phosphate, Carbamate von Aminosàuren, Ester und pharmakologisch verträglichen Salze.

Bevorzugt sind die Verbindungen, bei denen die Reste folgende Bedeutung annehmen:
- R¹: Wasserstoff, verzweigtes und unverzweigtes C₁-C₆-Alkyl, wobei ein C-Atom des Alkyl-Restes noch OR¹¹ oder eine Gruppe R⁵ tragen kann, wobei
- R¹¹: Wasserstoff oder C₁-C₄-Alkyl bedeutet, und
- R²: Wasserstoff, Chlor, Fluor, Brom, Iod, verzweigtes und unverzweigtes C₁-C₆-Alkyl, Nitro, CF₃, CN, NR²¹R²², NH-CO-R²³, OR²¹, wobei
- R²¹ und R²²: unabhängig voneinander Wasserstoff oder C₁-C₄-Alkyl bedeuten und
- R²³: Wasserstoff, C₁-C₄-Alkyl oder Phenyl bedeuten, und
- R³: -O-(CH₂)ₒ-(CHR³¹)ₘ-(CH₂)ₙ-R⁵, wobei
- R³¹: Wasserstoff, C₁-C₄-Alkyl, OH und O-C₁-C₄-Alkyl,
- m,o: unabhängig voneinander 0, 1 oder 2 bedeutet, und
- n: 1, 2, 3 oder 4 bedeutet, und
- R⁴: Wasserstoff, verzweigtes und unverzweigtes C₁-C₆-Alkyl, Chlor, Brom Fluor, Nitro, Cyano, NR⁴¹R⁴², NH-CO-R⁴³, OR⁴¹, wobei
- R⁴¹ und R⁴²: unabhängig voneinander Wasserstoff oder C₁-C₄-Alkyl bedeuten und
- R⁴³: C₁-C₄-Alkyl oder Phenyl bedeuten, und
- R⁵: NR⁵¹R⁵² oder einen der folgenden Reste 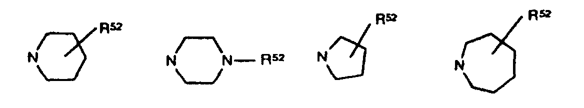 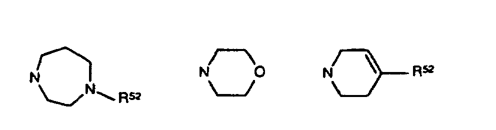 bedeutet, wobei
- R⁵¹: Wasserstoff und verzweigtes und unverzweigtes C₁-C₆-Alkyl bedeutet und
- R⁵²: Wasserstoff, verzweigtes und unverzweigtes C₁-C₆-Alkyl, Phenyl, 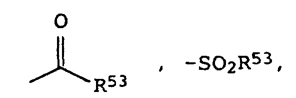
worin
R⁵³ verzweigtes oder unverzweigtes O-C₁-C₆-Alkyl, Phenyl, verzweigtes oder unverzweigtes C₁-C₄-Alkyl-Phenyl, wobei bei R⁵² und R⁵³ unabhängig voneinander ein Wasserstoff des C₁-C₆-Alkylrests durch einen der folgenden Reste substituiert sein kann: OH, O-C₁-C₄-Alkyl, Cyclohexyl, Cyclopentyl, Tetrahydronaphthyl, Cyclopropyl, Cyclobutyl, Cycloheptyl, Naphthyl und Phenyl, wobei die Carbocyclen der Reste R⁵² und R⁵³ unabhängig voneinander noch einen oder zwei der folgenden Reste tragen können: verzweigtes oder unverzweigtes C₁-C₆-Alkyl, verzweigtes oder unverzweigtes O-C₁-C₄-Alkyl, OH, F, Cl, Br, J, CF₃, NO₂, NH₂, CN, COOH, COOC₁-C₄-Alkyl, C₁-C₄-Alkylamino, CCl₃, C₁-C₄-Dialkylamino, SO₂-C₁-C₄-Alkyl, SO₂Phenyl, CONH₂, CONH-C₁-C₄-Alkyl, CONHPhenyl, CONH-C₁-C₄-Alkyl-Phenyl, NHSO₂-C₁-C₄-Alkyl, NHSO₂Phenyl, S-C₁-C₄-Alkyl, 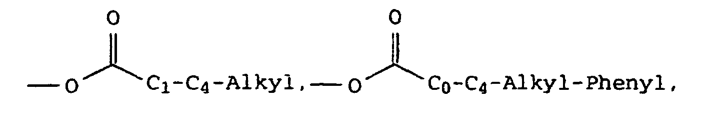 CHO, CH₂-O-C₁-C₄-Alkyl, -CH₂O-C₁-C₄-Alkyl-Phenyl, -CH₂OH, -SO-C₁-C₄-Alkyl, -SO-C₁-C₄-Alkyl-Phenyl, -SO₂NH₂, -SO₂NH-C₁-C₄-Alkyl und zwei Reste eine Brücke -O-(CH₂)_{1,2}-O- bilden, bedeutet.

Besonders bevorzugte Positionen für den Rest R² in der allgemeinen Formel I oder II sind die 3-Position und die 4-Position zum Benzimidazolring. Für den Rest R³ ist ebenfalls die 3-Position oder 4-Position zum Benzimidazolring bevorzugt.

Die besonders bevorzugte Bedeutung von R¹ ist Wasserstoff.

Die besonders bevorzugte Bedeutung von R² ist Wasserstoff, verzweigtes oder unverzweigtes C₁-C₆-Alkyl, Nitro, CN, NH₂, O-C₁-C₄-Alkyl.

Die besonders bevorzugte Bedeutung von R³ ist -O-(CH₂)p-R⁵ mit p gleich 2, 3 oder 4.
- R⁵: bedeutet bevorzugt einen 6-gliedrigen Ring, insbesondere Piperazin,
- R⁵²: bedeutet bevorzugt einen gegebenenfalls substituierten Phenylring, insbesondere falls R⁵ einen 6-gliedrigen Ring bedeutet.

Die besonders bevorzugte Bedeutung von R⁴ ist Wasserstoff.

Ganz besonders bevorzugt sind die jeweiligen Kombinationen der obigen bevorzugten Bedeutungen.

Bevorzugt sind außerdem Verbindungen mit folgenden Bedeutungen für die Substituenten:
- R¹: Wasserstoff, verzweigtes und unverzweigtes C₁-C₆-Alkyl, wobei ein C-Atom des Alkyl-Restes noch OR¹¹ oder eine Gruppe R⁵ tragen kann, wobei R¹¹ Wasserstoff oder C₁-C₄-Alkyl bedeutet, und
- R²: Wasserstoff, Chlor, Fluor, Brom, Jod, verzweigtes und unverzweigtes C₁-C₆-Alkyl, Nitro, CF₃, CN, NR²¹R²², NH-CO-R²³, OR²¹, wobei
R²¹ und R²² unabhängig voneinander Wasserstoff oder C₁-C₄-Alkyl bedeuten und
R²³ Wasserstoff, C₁-C₄-Alkyl oder Phenyl bedeuten, und
- R³: 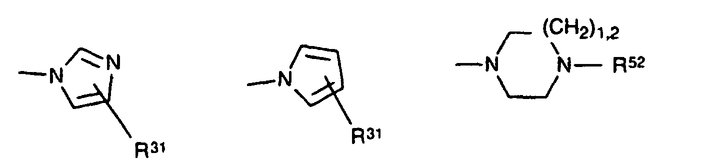 und
R³¹ Wasserstoff, CHO und -(CH₂)ₒ-(CHR³²)ₘ-(CH₂)ₙ-R⁵, wobei
R³² Wasserstoff, C₁-C₄-Alkyl, OH und O-C₁-C₄-Alkyl,
m,o unabhängig voneinander 0, 1 oder 2 bedeutet und
n 1, 2, 3 oder 4 bedeutet, und
- R⁴: Wasserstoff, verzweigtes und unverzweigtes C₁-C₆-Alkyl, Chlor, Brom Fluor, Nitro, Cyano, NR⁴¹R⁴², NH-CO-R⁴³, OR⁴¹,wobei
R⁴¹ und R⁴² unabhängig voneinander Wasserstoff oder C₁-C₄-Alkyl bedeuten und
R⁴³ C₁-C₄-Alkyl oder Phenyl bedeuten, und
- R⁵: NR⁵¹R⁵² oder einen der folgenden Reste 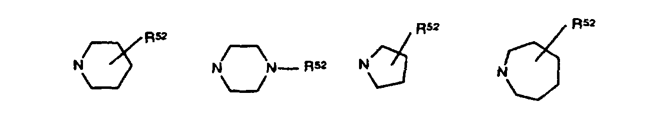 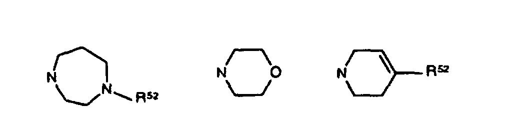 wobei
R⁵¹ Wasserstoff und verzweigtes und unverzweigtes C₁-C₆-Alkyl bedeutet und
R⁵² Wasserstoff, COCH₃, CO-O-C₁-C₄-Alkyl, COCF₃, verzweigtes und unverzweigtes C₁-C₆-Alkyl, wobei ein Wasserstoff des C₁-C₆-Alkylrests durch einen der folgenden Reste substituiert sein kann: OH, O-C₁-C₄-Alkyl und Phenyl und der Phenyl-Ring noch einen oder zwei der folgenden Reste tragen kann: Chlor, Brom, Fluor, verzweigtes und unverzweigtes C₁-C₄-Alkyl, Nitro, Amino, C₁-C₄-Alkylamino, C₁-C₄-Dialkylamino, OH, O-C₁-C₄-Alkyl, CN, SO₂-C₁-C₄-Alkyl, bedeutet.

Besonders bevorzugte Positionen für den Rest R² in der allgemeinen Formel I oder II sind die 3-Position und die 4-Position zum Benzimidazolring. Für den Rest R³ ist ebenfalls die 3-Position oder 4-Position zum Benzimidazolring bevorzugt.

Die besonders bevorzugte Bedeutung von R¹ ist Wasserstoff.

Die besonders bevorzugte Bedeutung von R² ist Wasserstoff, verzweigtes oder unverzweigtes C₁-C₆-Alkyl, Nitro, CN, NH₂, O-C₁-C₄-Alkyl. Besonders bevorzugt ist R² gleich Wasserstoff.

Für R³ gleich 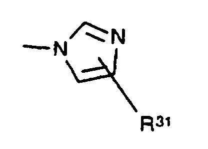 ist die besonders bevorzugte Bedeutung von R³¹ ist Wasserstoff oder -(CH₂)ₚ-R⁵, wobei
- p: 1 oder 2 bedeutet und
- R⁵²: Wasserstoff, verzweigtes und unverzweigtes C₁-C₆-Alkyl,
wobei ein Wasserstoff des C₁-C₆-Alkylrests durch einen der folgenden Reste substituiert sein kann: OH, O-C₁-C₄-Alkyl und Phenyl und der Phenyl-Ring noch einen oder zwei der folgenden Reste tragen kann: Chlor, Brom, Fluor, verzweigtes und unverzweigtes C₁-C₄-Alkyl, Nitro, Amino, C₁-C₄-Alkylamino, C₁-C₄-Dialkylamino, OH, O-C₁-C₄-Alkyl, CN, SO₂-C₁-C₄-Alkyl, bedeuten kann.

Für R³ gleich 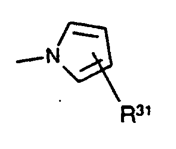 ist die besonders bevorzugte Bedeutung von R³¹ ist Wasserstoff oder -(CH₂)ₚ-R⁵, wobei
- p: 1 oder 2 bedeutet und
- R⁵²: Wasserstoff, verzweigtes und unverzweigtes C₁-C₆-Alkyl, wobei ein Wasserstoff des C₁-C₆-Alkylrests durch einen der folgenden Reste substituiert sein kann: OH, O-C₁-C₄-Alkyl und Phenyl und der Phenyl-Ring noch einen oder zwei der folgenden Reste tragen kann: Chlor, Brom, Fluor, verzweigtes und unverzweigtes C₁-C₄-Alkyl, Nitro, Amino, C₁-C₄-Alkylamino, C₁-C₄-Dialkylamino, OH, O-C₁-C₄-Alkyl, CN, SO₂-C₁-C₄-Alkyl, bedeuten kann.

Für R³ gleich 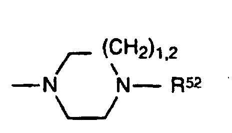 ist die besonders bevorzugte Bedeutung von
- R⁵²: Wasserstoff, verzweigtes und unverzweigtes C₁-C₆-Alkyl, wobei ein Wasserstoff des C₁-C₆-Alkylrests durch einen der folgenden Reste substituiert sein kann: OH, O-C₁-C₄-Alkyl und Phenyl und der Phenyl-Ring noch einen oder zwei der folgenden Reste tragen kann: Chlor, Brom, Fluor, verzweigtes und unverzweigtes C₁-C₄-Alkyl, Nitro, Amino, C₁-C₄-Alkylamino, C₁-C₄-Dialkylamino, OH, O-C₁-C₄-Alkyl, CN, SO₂-C₁-C₄-Alkyl, bedeuten kann.

Die besonders bevorzugte Bedeutung von R⁴ ist Wasserstoff.

Ganz besonders bevorzugt sind die jeweiligen Kombinationen der obigen bevorzugten Bedeutungen.

Die Verbindungen der Formel I können als Racemate, als enantiomerenreine Verbindungen oder als Diastereomere eingesetzt werden. Werden enantiomerereine Verbindungen gewünscht, kann man diese beispielsweise dadurch erhalten, daß man mit einer geeigneten optisch aktiven Base oder Säure eine klassische Racematspaltung mit den Verbindungen der Formel I oder ihren Zwischenprodukten durchführt.

Gegenstand der Erfindung sind auch zu Verbindungen der Formel I mesomere oder tautomere Verbindungen.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd. 10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxidund Tris.

Unter Prodrugs werden solche Verbindungen verstanden, die in vivo in Verbindungen der allgemeinen Formel I oder II metabolisiert werden. Typische Prodrugs sind Phosphate, Carbamate von Aminosäuren, Ester und andere.

Die Herstellung der erfindungsgemäßen 2-Phenylbenzimidazole der Formel I oder II kann auf verschiedenen Wegen erfolgen, die in folgenden Syntheseschemas skizziert wurden.

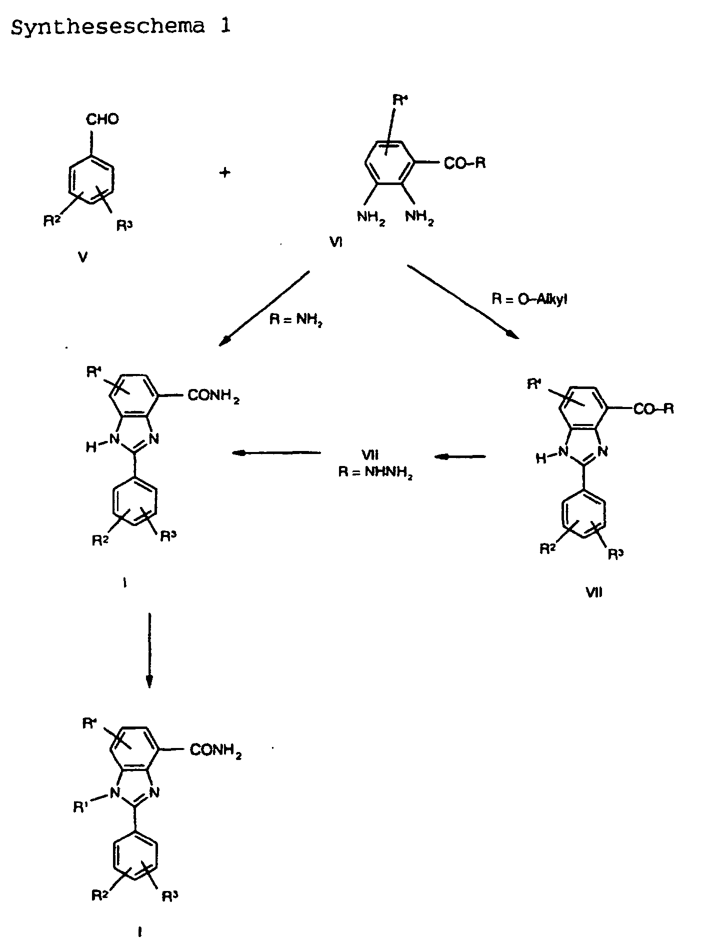

Durch Kondensation von Benzaldehyden V mit Phenylendiaminen VI erhält man das Benzimidazol VII, wobei man bevorzugt in polaren Lösungsmitten wie Ethanol oder Dimethylformamid mit Zusatz von Säuren wie Essigsäure bei erhöhter Temperatur arbeitet, in der Regel 80 bis 120°C. Günstig für die Reaktion ist der Zusatz von schwachen Oxidationsmittel wie Kupfer-II-Salzen, die als wäßrige Lösung zugesetzt werden.

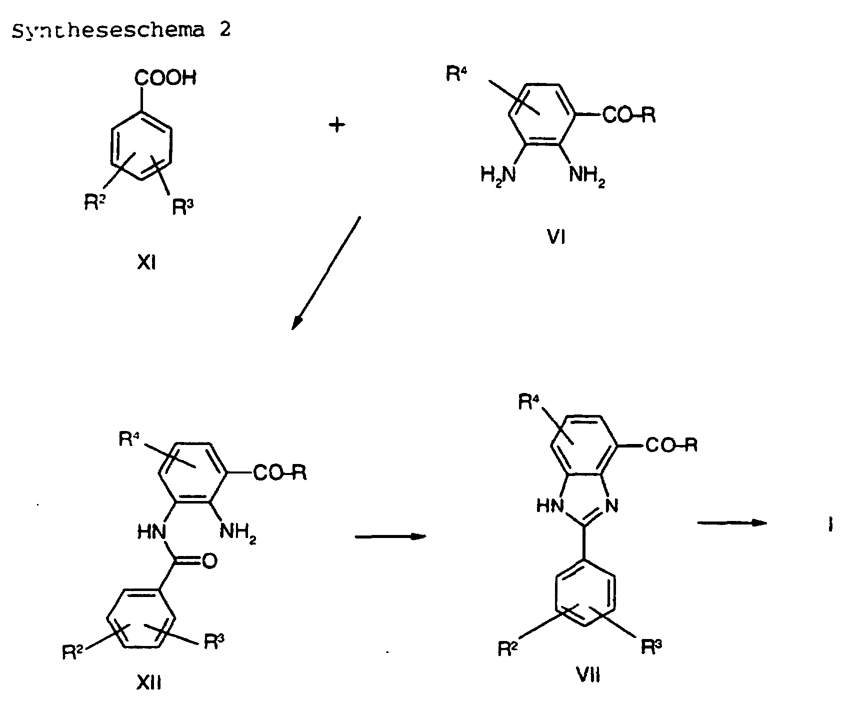

Wenn in dem Phenylendiamin VI R = NH₂ ist, entstehen bei der Kondensation direkt erfindungsgemäße Verbindungen I. Ansonsten kann man, falls R = O-Alkyl ist, diesen Ester mit Ammoniak, bei gegebenenfalls erhöhter Temperatur und erhöhtem Druck, zum Amid I umsetzen. Alternativ kann man den Ester XII mit Hydrazin in polaren Lösungsmitteln, wie die Alkohole Butanol und Ethanol oder auch Dimethylformamid, bei erhöhten Temperaturen, vorzugsweise 80 bis 130°C, umsetzen, wobei ein Hydrazid XII (R = NHNH₂) anfällt, das danach noch unter reduktiven Bedingungen, wie mit Raney-Nickel in Alkoholen unter Rückfluß, zum Amid I reduziert werden kann.

Eine Einführung des Restes R1 am Benzimidazol-Rest in I (R1 =H) gelingt unter üblichen Alkylierungsbedingungen, wie sie zum Beispiel in J.Het.Chem. 1995, 32, 707f und in Tetrahedron 1994, 50, 5535), wobei allerdings der Reaktionspartner R1-L (L = Abgangsgruppe wie Cl, Br. Und J) eingesetzt werden muß.

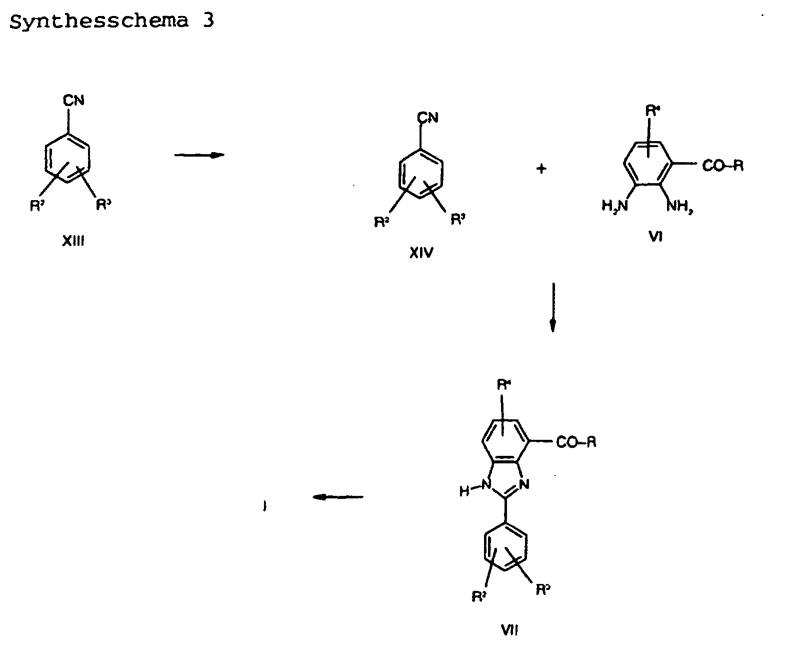

Alternativ zu den im Schema 1 gezeigten Benzaldehyden V kann man auch Benzoesäuren wie XI (siehe Schema 2) oder Benzonitrile wie XIII(siehe Schema 3) anstelle des Benzaldehyds einsetzen. Die Herstellung dieser Derivate erfolgt analog zur Herstellung der substituierten Benzaldehyde V. Ausgehend von XI erfolgt die Kondensation zu VII in zwei Stufen. Zuerst wird die Benzoesäure XI mit dem Anilin VI in einer peptidartigen Kupplung zum Amid XII umgesetzt. Dabei arbeitet man nach üblichen Bedingungen, die zum Beispiel im Houben-Weyl, Methoden der Organischen Chemie, 4. Aufl., E5, Kap.V bzw. C.R. Larock, Comprehensive Organic Transformations, VCH Publisher, 1989, Seite 972f. aufgelistet sind. Der Ringschluß erfolgt zum Benzimidazol erfolgt danach bei erhöhter Temperatur, zum Beispiel 60 bis 180°C, mit oder ohne Lösungsmitteln wie Dimethylformamid, unter Zusatz von Säuren wie Essigsäure oder direkt in Essigsäure selbst.

Die Reaktion des Phenylendiamins VI mit einem Benzonitril XIII erfolgt ebenfalls unter üblichen Bedingungen. Dabei kann man in Lösungsmitten wie Dimethylformamid unter Zusatz von Säuren bei erhöhter Temperatur, wie 60 bis 200°C, arbeiten. Allerdings kann man auch die üblichen Methoden zur Herstellung von Amidinen aus Benzonitrilen anwenden, wie sie in in Houben-Weyl, Methoden der organischen Chemie, E5, S. 1304 f., J. Amer.Chem.Soc. 1957, 427 und J.Org.Chem. 1987,1017 beschrieben sind.

Die vorliegende Erfindung betrifft außerdem 2,3-Diaminobenzamide der Formel XX, XXI und deren Synthese und Verwendung als Zwischenstufen.

Diaminobenzamide, die am Amid-Rest eine substituierte Alkylkette tragen, werden in WO 9631462 zur Behandlung neurodegenerativer Krankheiten beschrieben. Diaminobenzamide, die am Amid-Rest einen substituierten Aryl-Rest tragen, werden in JP 09059236 zur Behandlung von Entzündungen und Allergien beschrieben. Die Effekte von Benzohydroxamsäuren auf die DNA-Synthese wurden in *Bull. Soc. Chim. Belg*. 1997, *106*, 767 untersucht.

Amino-dibenzodiazepinone wurden in P. V. Khadikar et al., *J. Heterocycl. Chem.* 1998, *35*, 675 hergestellt. Die Synthese von 2-Phenyl-benzimidazyl-4-amiden ist in *J. Chem. Soc. Perkin Trans 1*, 1979, 2302-2307 beschrieben worden. Analoge Verbindungen, die am Amid-Rest noch eine substituierte Alkyl-Kette tragen, und die cytotoxische Wirkung haben sollen, sind in *J. Med. Chem*. 1990, *33*, 814-819 aufgeführt. In WO 97/04771 sind Benzimidazol-4-amide aufgeführt, die das Enzym PARP hemmen. Insbesondere sind Derivate als wirksam beschrieben, die einen Phenylring in 2-Stellung tragen, wobei der Phenyl-Ring noch mit einfachen Substituenten wie Nitro, Methoxy und CF₃ substituiert sein kann.

Zur Veranschaulichung der Synthesestrategie in WO 97/04771 ist in Schema 4 beispielhaft die Synthese von 2-Phenylbenzimidazol-4-carboxamid (NU 1070) dargestellt.

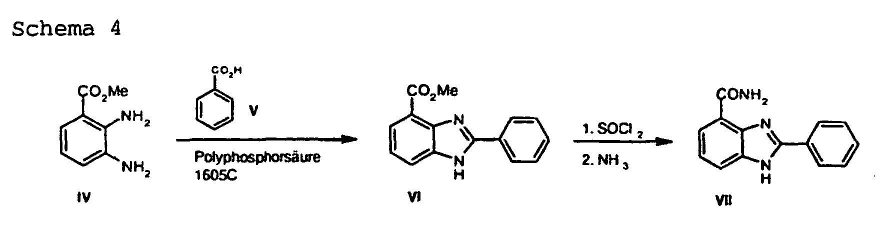

Die Umsetzung von Diaminobenzoesäuremethylester IV mit Benzoesäure V in Polyphosphorsäure ergibt den Benzimidazol-4-carbonsäureester VI in 20 % Ausbeute. Abschließend wird Ester VI mittels Säurechloridbildung in das Amid VII überführt. Für diesen Schritt geben die Autoren eine Ausbeute von 62 % an. Für die Synthesesequenz ergibt sich eine Gesamtausbeute von 12 %. Die Gesamtausbeuten für die Synthesen aller anderen in WO 97/04771 genannten Beispiele liegen in einem Rahmen von 5 bis 19 %. Ein großer Nachteil dieser Synthesestrategie ist die Tatsache, daß jede zu VI analoge Verbindung noch in das Amid überführt werden muß, das erst den wirksamen PARP-Inhibitor darstellt.

Gegenstand der vorliegenden Erfindung sind 2,3-Diamino-benzamide der Formel XX und XXI: 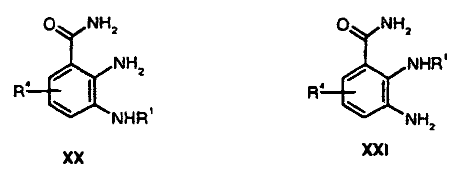 worin

R⁴ und R¹ die oben genannten Bedeutungen besitzen, sowie deren Salze.

Die Synthese der Verbindungen XX bzw. XXI erfolgt nach Schema 5 durch Hydrazinolyse eines geeignet substituierten Esters VIII mit Hydrazinhydrat in einem Alkohol wie n-Butanol bei 100°C und anschließender Reduktion des Hydrazids mit Raney-Nickel in polaren Lösungsmitteln wie Dimethylformamid bei 100°C.

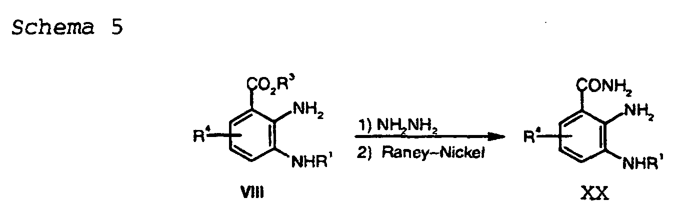

Überraschender Weise ergaben sich in den Synthesen von Benzimidazol-4-amiden aus den Verbindungen XX bzw. XXI außerdem höhere Gesamtausbeuten als die in WO 97/04771 beschriebenen.

Die Synthese von Benzimidazol-4-amiden aus den Verbindungen der Formel XX und XXI wird in Schema 6 bzw. Schema 7 beschrieben.

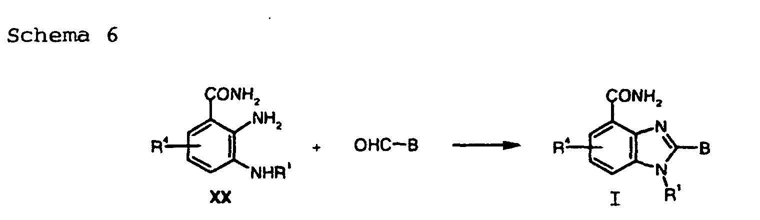

Durch Kondensation eines geeigneten Aldehyds OHC-B mit Verbindungen XX bzw. XXI erhält man das Benzimidazol I, wobei man bevorzugt in polaren Lösungsmitteln wie Ethanol oder Dimethylformamid und Zusatz von Säuren wie Essigsäure bei erhöhter Temperatur arbeitet, in der Regel 80 bis 120°C. Günstig für die Reaktion ist der Zusatz von schwachen Oxidationsmitteln wie Kupfer-II-Salzen, die als wäßrige Lösung zugesetzt werden.

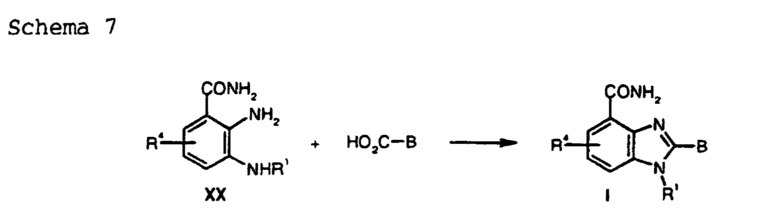

Mit geeigneten Säuren HOOC-B erfolgt zunächst eine peptidartige Kupplung mit den Verbindungen XX bzw. XXI. Dabei arbeitet man nach üblichen Bedingungen, die zum Beispiel in Houben Weyl, Methoden der Organischen Chemie, 4. Aufl., E5, Kap. V bzw. C.R. Larock, Comprehensive Organic Transformations, VCH Publisher, 1989, S. 972f. aufgelistet sind. Der Ringschluß erfolgt anschließend bei erhöhter Temperatur, zum Beispiel 60 bis 180°C, mit oder ohne Lösungsmitteln wie Dimethylformamid, unter Zusatz von Säuren wie Essigsäure oder direkt in Essigsäure selbst.

Zum Vergleich der Gesamtausbeuten der neuen Synthesestrategie mit denen in WO 97/04771 ist die Synthese von 2-Phenylbenzimidazol-4-carboxamid in Schema 11 dargestellt. Die Umsetzung von Ester XIV zum Amid XV erfolgt in 70 %. Die Synthese des Benzimidazols VII durch Kondensation von XV mit Benzaldehyd XVI mit anschließender Oxidation verläuft mit 85 % Ausbeute. Die daraus resultierende Gesamtausbeute von 60 % übertrifft die entsprechende Gesamtausbeute von 12 % in WO 97/04771.

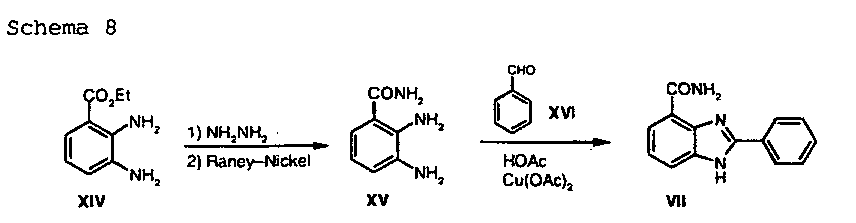

Die in der vorliegenden Erfindung enthaltenen substituierten 2-Phenylbenzimidazole I oder II stellen Inhibitoren des Enzyms Poly-(ADP-ribose)polymerase oder PARP (EC 2.4.2.30) dar. Die inhibitorische Wirkung der substituierten 2-Phenylbenzimidazole I oder II wurde mit einem in der Literatur bereits bekannten Enzymtest ermittelt, wobei als Wirkmaßstab ein Kᵢ-Wert ermittelt wurde. Die 2-Phenylbenzimidazole I wurden in dieser Weise auf Hemmwirkung des Enzyms Poly(ADP-ribose)polymerase oder PARP - (EC 2.4.2.30) gemessen.

### Bestimmung des Wasserlöslichkeit

Eine zu messende Verbindung wird direkt in einem festgelegten Volumen Wasser gelöst und die entstandene Lösung mit einer Natriumacetat-Lösung auf pH 5 bis 6 eingestellt, so daß die zu prüfende Konzentration des Wirkstoffs erreicht wird. Falls die Meßsubstanz nicht als wasserlösliches Salz vorliegt, wurde diese in möglichst wenig Dimethylsulfoxid gelöst und anschließend mit Wasser verdünnt (Endkonzentration an Dimethylsulfoxid ≤ 1 %), wonach auch hier der pH-Wert noch eingestellt wurde. Der potente PARP-Inhibitor NU 1076 (WO 97/04771) zeigte hier eine Löslichkeit < 0,01 %, wogegen das erfindungsgemäße Beispiel 2 eine Löslichkeit > 0,5 % aufweist.

Die substituierten 2-Phenylbenzimidazole der allgemeinen Formeln I stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten Enzymaktivität dieser Enzyme verbunden sind, dienen.

Die Verbindungen der Formeln I können zur Herstellung von Arzneimitteln zur Behandlung von Schädigungen nach Ischämien und zur Prophylaxe bei erwarteten Ischämien verschiedener Organe eingesetzt werden.

Die vorliegenden 2-Phenylbenzimidazole der allgemeinen Formel I können danach zur Behandlung und Prophylaxe von neurodegenerativen Krankheiten, die nach Ischämie, Trauma (Schädel-Hirntrauma), Massenblutungen, Subarachnoidal-Blutungen und Stroke auftreten, und von neurodegenerativen Krankheiten, wie multipler Infarkt-Dementia, Alzheimer Krankheit, Huntington Krankheit und von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, des akuten Nierenversagens oder von Schädigungen, die während und nach einer Nierentransplantation auftreten, dienen. Weiterhin können die Verbindungen der allgemeinen Formel I zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator), und von Mikroinfarkten während und nach Herzklappenersatz, Aneurysmenresektionen und Herztransplantationen dienen. Ebenfalls können die vorliegenden 2-Phenylbenzimidazole I zur Behandlung einer Revascularisation kritisch verengter Koronaraterien, zum Beispiel bei der PCTA und Bypass-Operationen, und kritisch verengter peripherer Arterien, zum Beispiel Beinarterien, dienen. Zudem können die 2-Phenylbenzimidazole I bei der Chemotherapie von Tumoren und deren Metastasierung nützlich sein und zur Behandlung von Entzündungen und rheumatischen Erkrankungen, wie z.B. rheumatischer Arthritis, dienen.

Neue PARP-Inhibitoren können in relevanten pharmakologischen Modellen auf ihre therapeutische Wirksamkeit überprüft werden. Beispiele für einige geeignete Modelle sind dazu in Tabelle 1 aufgeführt.

**Tabelle 1**

| Krankheit | Modell | Literatur |
|---|---|---|
| Neurodegenerative Erkrankungen (Schlaganfall, Parkinson etc.) | NMDA-Exzitotoxizität in der Maus oder Ratte | |
| Schlaganfall | Permanente MCAO("middle cerebral artherial occlusion") | Tokime T. et al., J. Cereb Blood Flow Ketab, 18(9):991-7, 1998 |
| | | Guegan C. Brain Research. Molecular Brain Research 55(1) 133-40, 1998 |
| | Transiente, fokale MCAO in ratte oder Maus | Eliasson MJL et al., Nat Med 1997, 3:1089-1095. |
| | | Endres M. et al., J. Cereb Blood Flow Metab 1997, 17:1143-1151. |
| | | Takahashi K. et al., J. Cereb Blood Flow Metab 1997, 17:1137-1142. |
| Parkinsonsche Krankheit | MPTP (1-Methyl-4-phenyl-1,2,3,6-tetrahydro-pyridin) Toxizität in der Maus/Ratte | Cosi C. et al., Brain Res. , 1998 809(1):58-67. |
| | | Cosi C. et al. , Brain Res., 1996 729(2):264-9. |
| Herzinfarkt | Koronargefäß-Okklusion an Ratte, Schwein oder Kaninchen | Richard V. et al., Br. J. Pharmacol 1994, 113, 869-876. |
| | | Thiemermann C. et al., Proc Natl Acad Sci USA. 1997, 94(2):679-83. |
| | | Zingarelli B. et al., Cardiovasc Res. 1997, 36(2):205-15. |
| | Langendorfherz-modell an Ratte oder Kaninchen | Beschreibung siehe unten |
| Septischer Schock | Endotoxin Schock in der Ratte | Szabo C. et al., J. Clin Invest, 1997, 100(3):723-35. |
| | Zymosan oder Carrageenan induziertes multiples Organversagen in Ratte oder Maus | Szabo C. et al. J. Exp Med. 1997, 186(7):1041-9. |
| | | Cuzzocrea S. et al. Eur J. Pharmacol. 1998, 342(1):67-76. |
| Rheumatoide Arthritis | Adjuvant oder Collagen induzierte Arthritis in Ratte oder Maus | Szabo C. et al., Proc Natl Acad Sci USA. 1998, 95(7):3867-72. |
| Diabetes | Streptozotocin und Alloxan induiziert bzw. Obesity assoziert | Uchigata Y. et al., Diabetes 1983, 32: 316-318. Masiello |
| | | P. et al., Dia-betologia 1985, 28: 683-686. |
| | | Shimabukuro M. et al., J. Clin Invest 1997, 100: 290-295. |
| Krebs | | Schlicker et al. 1999 75:1, 91-100 |

Die erfindungsgemäßen Arzneimittelzubereitungen enthalten neben den üblichen Arzneimittel-Hilfsstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-%, enthalten.

Bei der inneren Anwendung werden die Präparationen in Einzeldosen verabreicht. In einer Einzeldosis werden pro kg Körpergewicht 0,1 bis 100 mg gegeben. Die Zubereitungen können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiel 1

### 2(4(2-(N,N-Diethylamino)eth-1-yloxy)phenyl)-benzimidazol-4-carbonsäureamid

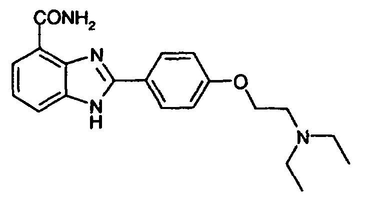

a) 4(2-(N,N-Diethylaminoeth-1-yloxy)-benzaldehyd
   15 g (122 mMol) 4-Hydroxybenzaldehyd, 16,7 g (122 mMol) N(2-Chlorethyl)-N,N-diethylamin und 33,9 g (246 mMol) Kaliumkarbonat wurden zusammnen mit einer Spatelspitze 18-Krone-6 in 300 ml Ethylmethylketon für 6 Stunden unter Rückfluß gekocht. Anschließend wurde filtriert und das Filtrat im Vakuum eingeengt. Der Rückstand wurde zwischen Ether und 2 M Natronlauge verteilt, die Ether-Phase abgetrennt, getrocknet und im Vakuum eingeengt. Man erhielt 24,8 g des Zwischenproduktes.
b) 2(4(2-(N,N-Diethylamino)eth-1-yloxy)phenyl)-benzimidazol-4-carbonsäureethylester
   2 g (11 mMol) 2,3-Diaminobenzoesäureethylester und 1,4 ml konzentrierte Essigsäure wurden in 25 ml Methanol gelöst. Anschließend wurden 3,2 g (14,4 mMol) der Zwischenverbindung 1a, gelöst in 50 ml Methanol, innerhalb von 30 Minuten zugetropft. Danach wurden 2,9 g (14,4 mMol) Kupfer-II-acetat, gelöst in 37,5 ml warmen Wasser, zügig zugetropft und anschließend alles für 20 Minuten unter Rückfluß gekocht. Man kühlte die Reaktionslösung auf 50°C und gab 4,5 ml 32%iger Salzsäure zu. Danach tropfte man noch vorsichtig eine Lösung aus 4,3 g Natriumsulfid-Hydrat in 25 ml Wasser zu und rührte alles noch für 15 Minuten. Die Reaktionslösung wurde auf Eiswasser gegossen und der anfallende Niederschag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 4,4 g des Zwischenproduktes.
c) 2(4(2-(N,N-Diethylamino)eth-1-yloxy)phenyl)-benzimidazol-4-carbonsäurehydrazid
   Zu 4,1 g (10,7 mMol) der Zwischenverbindung 1b in 30 ml Ethanol wurden 2,7 g (54 mMol) hydrazinhydrat gegeben und alles für 10 Stunden unter Rückfluß gekocht. Anschließend wurde das organische Lösungsmittel im Vakuum entfernt und der Rückstand zwischen Wasser und Essigester verteilt. Die Essigester-Phase wurde abgetrennt, getrocknet und im Vakuum eingeengt. Der so erhaltene Rückstand wurde noch mit Ether behandelt und erneut abgesaugt, wonach man 1.7g der Zwischenverbindung.
d) 2(4(2-(N,N-Diethylamino)eth-1-yloxy)phenyl)-benzimidazol-4-carbonsäureamid
   Zu 1,6 g (4,5 mMol) der Zwischenverbindung 1c in 45 ml Dimethylformamid/Wasser (2/1) wurden ca. 1,6 g Raney-Nickel gegeben und alles für 6 Stunden auf 100°C erwärmt. Anschließend wurde das Reaktionsgemisch filtriert und das Filtrat mit viel Wasser verdünnt, wobei das Produkt ausfiel. Man erhielt 1,2 g des Produktes.
   ¹H-NMR (D₆-DMSO). δ = 0,95(6H), 2,6(4H), 2,8(2H), 4,1(2H), 7,1(2H), 7,3(1H), 7,7(1H + NH), 7,85(1H), 8,2(2H) und 9,4 (NH)ppm.

### Beispiel 2

### 2(4(2-(N,N-Diethylamino)eth-1-yloxy)phenyl)-benzimidazol-4-carbonsäureamid x 2 Hydrochlorid

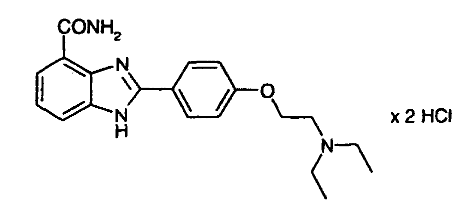

0,2g des Produktes aus Beispiel 1 wurden in einem Gemisch aus Essigester und wenig Tetrahydrofuran gelöst und mit etherischer Chlorwasserstoff-Lösung versetzt, wobei sich ein Niederschlag bildet. Dieser Niederschlag wurde abgesaugt, mit Aceton aufgeschlämmt und erneut abgesaugt, wonach man ca. 200 mg des Produktes erhielt.
¹H-NMR (D₆-DMSO). δ = 1,2(6H), 3,2(4H), 3,3(2H), 4,5(2H), 7,25(1H), 7,4(1H), 7,8-7,9(2H), 8,3(2H), 9,0(NH) und 10,5 (NH) ppm.

### Beispiel 3

### 2(3(2-(N,N-Diethylamino)eth-1-yloxy)phenyl)-benzimidazol-4-carbonsäureamid

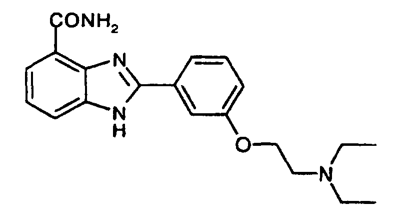

a) 3(2-(N,N-Diethylaminoeth-1-yloxy)-benzaldehyd
   6,1 g (50 mMol) 3-Hydroxybenzaldehyd wurden in 100 ml Ethanol gelöst und 3,5 g (50 mMol) Natriumethanolat zugegeben. Man rührte alles für 15 Minuten. Danach wurden 7,5 g (55 mMol) N(2-Chlorethyl)-N,N-diethylamin zugefügt und alles für 12 Stunden unter Rückfluß gekocht. Anschließend wurde das Reaktionsgemisch im Vakuum eingeengt. Der Rückstand wurde daanch zwischen Ether und 1M Natronlauge verteilt, die Ether-Phase abgetrennt, getrocknet und im Vakuum eingeengt. Man erhielt 7.6g des Zwischenproduktes.
b) 2(3(2-(N,N-Diethylamino)eth-1-yloxy)phenyl)-benzimidazol-4-carbonsäureethylester
   1 g (5,5 mMol) 2,3-Diaminobenzoesäureethylester und 0,68 ml konzentrierte Essigsäure wurden in 20 ml Methanol gelöst. Anschließend wurden 1,6 g (7,2 mMol) der Zwischenverbindung 3a, gelöst in 30 ml Methanol, innerhalb von 30 Minuten zugetropft. Danach wurden 1,1 g (5,5 mMol) Kupfer-II-acetat, gelöst in 19 ml warmen Wasser, zügig zugetropft und anschließend alles für 20 Minuten unter Rückfluß gekocht. Man kühlte die Reaktionslösung auf 50°C und gab 2,25 ml 32%iger Salzsäure zu. Danach tropfte man noch vorsichtig eine Lösung aus 2,13 g Natriumsulfid-Hydrat in 15 ml Wasser zu und rührte alles noch für 15 Minuten. Die Reaktionslösung wurde auf Eiswasser gegossen und der anfallende Niederschag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 2,4 g des Zwischenproduktes.
c) 2(3(2-(N,N-Diethylamino)eth-1-yloxy)phenyl)-benzimidazol-4-carbonsäurehydrazid
   Zu 2,3 g (6,0 mMol) der Zwischenverbindung 3b in 30 ml Butanol wurden 1,5 g (30 mMol) hydrazinhydrat gegeben und alles für 10 Stunden auf 120°C erwärmt. Anschließend wurde das Reaktionsgemisch mit viel Wasser verdünnt und mit Essigester extrahiert. Die Essigester-Phase wurde abgetrennt, getrocknet und im Vakuum eingeengt. Man erhielt 1,7 g der Zwischenverbindung.
d) 2(3(2-(N,N-Diethylamino)eth-1-yloxy)phenyl)-benzimidazol-4-carbonsäureamid
   Zu 1 g (2,7 mMol) der Zwischenverbindung 3c in 30 ml Dimethylformamid/Wasser (2/1) wurden ca. 1,5 g Raney-Nickel gegeben und alles für 6 Stunden auf 100°C erwärmt. Anschließend wurde das Reaktionsgemisch filtriert und das Filtrat mit viel Wasser verdünnt, wobei das Produkt ausfiel. Man erhielt 0,74 g des Produktes.
   ¹H-NMR (D₆-DMSO). δ = 1,0(6H), 2,6(4H), 2,9(2H), 4,15(2H), 7,1(1H), 7,4(1H), 7,5(1H), 7,7-7,9 (5H) und 9,3 (NH) ppm.

### Beispiel 4

### 2(3(2-(N,N-Diethylamino)eth-1-yloxy)phenyl)-benzimidazol-4-carbonsäureamid x 2 Hydrochlorid

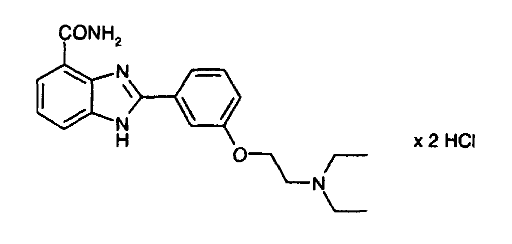

0,2 g des Produktes aus Beispiel 3 wurden in einem Gemisch aus Essigester und Tetrahydrofuran gelöst und mit etherischer Chlorwasserstoff-Lösung versetzt, wobei sich ein Niederschlag bildet. Dieser Niederschlag wurde abgesaugt, mit Aceton aufgeschlämmt und erneut abgesaugt, wonach man ca. 200 mg des Produktes erhielt.
¹H-NMR (D₆-DMSO). δ = 1,3(6H), 3,2(4H), 3,6(2H), 4,6(2H), 7,2-8,1(8H), 9,0(1H), und 10,8(NH) ppm.

Analog dem Beispiel 1 wurden hergestellt:

### Beispiel 5

### 2(3(2-(N,N-Dimethylamino)eth-1-yloxy)phenyl)-benzimidazol-4-carbonsäureamid

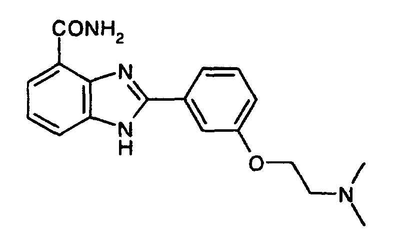

¹H-NMR (D₆-DMSO). δ = 2,2(6H), 2,7(2H), 4,2(2H), 7,0-8,0(9H) und 9,3(1H) ppm.

### Beispiel 6

### 2(3(2-(N,N-Dimethylamino)eth-1-yloxy)-4-methoxy-phenyl)-benzimidazol-4-carbonsäureamid

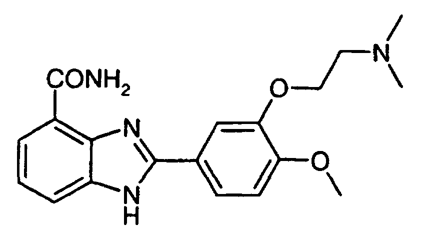

¹H-NMR (D₆-DMSO). δ = 2,25(6H), 2,75(2H), 3,8(3H), 4,1(2H), 7,0-8,1(8H) und 9,4(1H) ppm.

### Beispiel 7

### 2(3(2-(N,N-Dimethylamino)eth-1-yloxy)-4-methoxy-phenyl)-benzimidazol-4-carbonsäureamid x 2 HCl

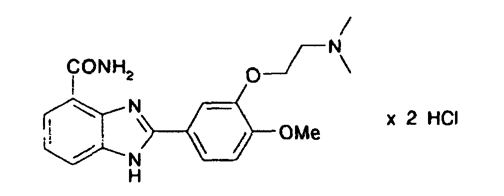

¹H-NMR (D₂O): δ = 3,0(6H), 3,7(2H), 3,8(3H), 4,3(2H), 6,9(1H), 7,3(1H), 7,3-7,5(3H) und 7,7(3H) ppm.

### Beispiel 8

### 2(2(2-(N,N-Dimethylamino)eth-1-yloxy)-phenyl)-benzimidazol-4-carbonsäureamid x 2 HCl

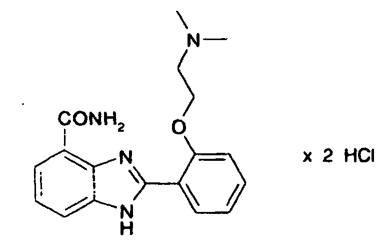

¹H-NMR (D₆-DMSO): δ = 2,9(6H), 3,7(2H), 4,7(2H), 7,2-8,3(8H), 8,9(breit) und ca 11(breit) ppm.

### Beispiel 9

### 2(3(2-(N,N-Dimethylamino)eth-1-yloxy)-phenyl)-benzimidazol-4-carbonsäureamid x 2 Hydrochlorid

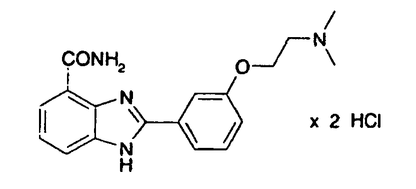

¹H-NMR (D₆-DMSO): δ = 2,9(6H), 3,5(2H), 4,5(2H), 7,2-8,1(8H), 9,0(breit) und ca 10,8(breit) ppm.

### Beispiel 10

### 2(3(3-(tert.-Butoxycarbonylamino)prop-1-yloxy)-phenyl)-benzimidazol-4-carbonsäureamid

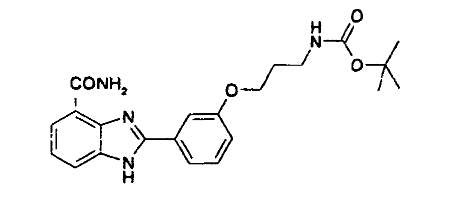

¹H-NMR (D₆-DMSO): δ = 1,3(9H), 1,9(2H), 3,1(2H), 4,1(2H), 6,9-8,0(9H) und ca 9,3(breit) ppm.

### Beispiel 11

### 2(3(3-(tert.-Butoxycarbonylamino)eth-1-yloxy)-phenyl)-benzimidazol-4-carbonsäureamid

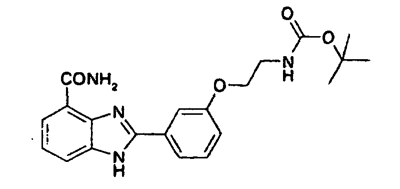

¹H-NMR (D₆-DMSO): δ = 1,3(9H), 3,3(2H), 4,1(2H), 7,0-8,0(9H) und ca 9,3(breit) ppm.

### Beispiel 12

### 2(3(3-(4(3-Chlorphenyl)piperazin-1-yl)prop-1-yloxy)-phenyl)-benzimidazol-4-carbonsäureamid

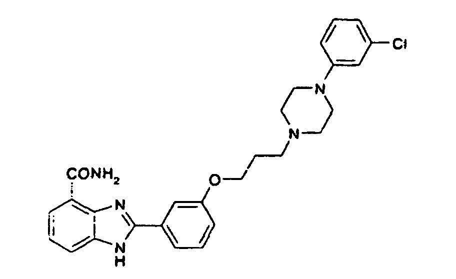

¹H-NMR (D₆-DMSO): δ = 2,3(2H), 3,3-3,5(6H), 3,7(2H), 3,7-4,3(6H), 6,9-8,0(11H), 9,1(breit) und ca 10,9(breit) ppm.

### Beispiel 13

### 2(3(3-(N,N-Diethylamino)prop-1-yloxy)-phenyl)-benzimidazol-4-carbonsäureamid x 2 HCl

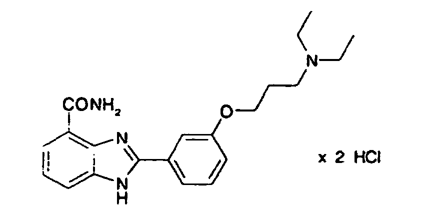

¹H-NMR (D₆-DMSO): δ = 1,2(6H), 2,2(2H), 3,2(4H), 3,8(2H), 4,3(2H), 7,1-8,0(7H), 9,1(breit) und ca 10,5(breit) ppm.

### Beispiel 14

### 2(3(3-Aminoprop-1-yloxy)-phenyl)-benzimidazol-4-carbonsäureamid x 2HCl

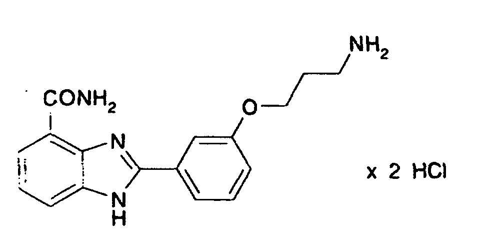

¹H-NMR (D₆-DMSO): δ = 2,1(2H), 3,0(2H), 4,2(2H), 7,2(1H), 7,5(2H), 7,8-8,1(6H), 8,2(breit) und ca 8,9(breit) ppm.

### Beispiel 15

### 2(3(2-Aminoeth-1-yloxy)-phenyl)-benzimidazol-4-carbonsäureamid x 2HCl

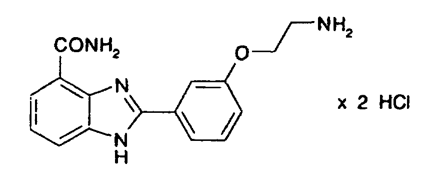

¹H-NMR (D₆-DMSO): δ = 3,2(2H), 4,2(2H), 7,1-8,0(9H), 8,2(breit) und 9,0(breit) ppm.

Folgende Beispiele können analog der obigen Vorschriften hergestellt werden.

### Beispiel 16

### 2(4(3-(N,N-Diethylamino)prop-1-yloxy)-phenyl)-benzimidazol-4-carbonsäureamid x 2 HCl

¹H-NMR (D₆-DMSO): δ = 1,3(6H), 2,2(2H), 3,2(6H), 4,2(2H), 7,2(2H), 7,5(1H), 7,8-8,0(3H), 8,35(2H), 8,9(1H) und 10,7(breit) ppm.

### Beispiel 17

### 1-(3(N,N-Diethylamino)-prop-1-yl)-2(4(3-(N,N-diethylamino)prop-1-yloxy)-phenyl)-benzimidazol-4-carbonsäureamid x 2 HCl

¹H-NMR (D₆-DMSO): δ = 1,1-1,3(12H), 2,2(4H), 2,9-3,3(12H), 4,2(2H), 4,5(2H), 7,2(2H), 7,6(1H), 7,8-8,1(3H), 8,3(1H), 8,4(1h), 8,9(1H)und 11,0(breit) ppm.

### Beispiel 18

### 2(4(2-(Pyrrolidin-1yl)-eth-1-yloxy)-phenyl)-benzimidazol-4-carbonsäureamid x 2 HCl

¹H-NMR (D₆-DMSO): δ = 1,3(1H), 1,7-2,0(5H), 3,0(2H), 3,5(4H), 4,5(2H), 7,2(2H), 7,3(1H), 7,7-8,0(3H), 8,2(2H), 8,9(breit) und 10,7(breit) ppm.

### Beispiel 19

### 1-(3(Pyrrolidin-1-yl)-prop-1-yl)-2(4(2-(pyrrolidin-1yl)-eth-1-yloxy)-phenyl)-benzimidazol-4-carbonsäureamid x 2 HCl

¹H-NMR (D₆-DMSO): δ = 1,3(2H), 1,7-1,9(10H), 3,0(4H), 3,3-3,6(8H), 4,5(2H), 4,9(2H), 7,1(2H), 7,5(1H), 7,7-8,0(3H), 8,1(2H), 9,0(breit), 10,8(breit) und 11,2(breit) ppm.

### Beispiel 20

### 2(4(3 (N,N-Benzylmethylamino)-prop-1-yloxy)-phenyl)-benzimidazol-4-carbonsäureamid x 2 HCl

### Beispiel 21

### 1(3(N,N-Benzylmethylamino)-prop-1-yl)-2(4(3(N,N-benzylmethylamino)-prop-1-yloxy)-phenyl)-benzimidazol-4-carbonsäureamid x 2 HCl

MS: m/e = 575 (M⁺).

### Beispiel 22

### 2(4(3(4-Methylpiperazin-1-yl)-prop-1-yloxy)-phenyl)-benzimidazol-4-carbonsäureamid x 3 HCl

MS: m/e = 393 (M⁺).

### Beispiel 23

### 2(3(2(N,N-Benzylmethylamino)-eth-1-yloxy)-4-nitrophenyl)-benzimidazol-4-carbonsäureamid

¹H-NMR (D₆-DMSO): δ = 1,0(6H), 2,5-2,8(4H), 2,9(2H), 4,3(2H), 7,3(1H), 7,8-8,2(6H) und 9,1(1H) ppm.

### Beispiel 24

### 2(4(3-Trifluoracetamidomethyl-pyrrol-1-yl)phenyl)-benzimidazol-4-carbonsäureamid

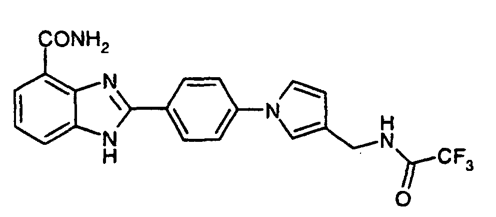

a) 2 (4-Nitrophenyl)-benzimidazol-4-carbonsäureethylester
   1,5 g (8,3 mMol) 2,3-Diaminobenzoesäureethylester und 1,1 ml konzentrierte Essigsäure wurden in 50 ml Methanol gelöst. Anschließend wurden 1,6 g (10,8 mMol) 4-Nitrobenzaldehyd, gelöst in 150 ml Methanol, innerhalb von 30 Minuten zugetropft. Danach wurden 2,2 g (10,8 mMol) Kupfer-II-acetat, gelöst in 100 ml warmen Wasser, zügig zugetropft und anschließend alles für 20 Minuten unter Rückfluß gekocht. Man kühlte die Reaktionslösung auf 50°C und gab 3 ml 32%iger Salzsäure zu. Danach tropfte man noch vorsichtig eine Lösung aus 3,1 g Natriumsulfid-Hydrat in 50 ml Wasser zu und rührte alles noch für 15 Minuten. Die Reaktionslösung wurde auf Eiswasser gegossen und der entstandene Niederschlag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 2,2 g des Zwischenproduktes.
b) 2(4(4-Nitro-phenyl)-benzimidazol-4-carbonsäurehydrazid
   Zu 2,1 g (6,7 mMol) der Zwischenverbindung 24a in 25 ml Ethanol wurden 1,7 ml (34 mMol) Hydrazinhydrat gegeben und alles für 4 Stunden unter Rückfluß gekocht. Anschließend wurde das organische Lösungsmittel im Vakuum entfernt und der Rückstand zwischen Wasser und Essigester verteilt. Die Essigester-Phase wurde abgetrennt, getrocknet und im Vakuum eingeengt. Der so erhaltene Rückstand wurde noch mit Ether behandelt und erneut abgesaugt, wonach man 1,7 g der Zwischenverbindung erhielt.
c) 2(4(4-Amino-phenyl)-benzimidazol-4-carbonsäureamid
   Zu 1,7 g (5,7 mMol) der Zwischenverbindung 24b in 120 ml Ethanol/Essigsäure (5/1) wurden ca. 1 g Palladium-Kohle (10%ig) und alles mit Wasserstoff hydriert. Anschließend wurde das Reaktionsgemisch filtriert und im Vakuum eingeengt. Der Rückstand wurde in 70 ml eines Gemisches aus Dimethylformamid und Wasser (7/3) gelöst. Anschließend wurden 2 g Raney-Nickel zugegeben und alles für 4 h auf 100°C erwärmt. Danach wurde das Reaktionsgemisch filtriert und das Filtrat im Vakuum eingeengt. Der so erhaltene Rückstand wurde in Ether dispergiert und abgesaugt, wobei man 1,5 g des Produktes erhielt.
d) 2(4(3-Trifluoracetamidomethyl-pyrrol-1-yl)phenyl)-benzimidazol-4-carbonsäureamid
   1,4 g (5,6 mMol) der Zwischenverbindung 24c und 1,8 g (6,9 mMol) 2,5-Dimethoxy-3-(trifluoracet-amidomethyl)tetrahydrofuran wurden in 50 ml konzentrierter Essigsäure gegeben und für 10 Minuten unter Rückfluß erwärmt. Anschließend wurde alles im Vakuum eingeengt und der anfallende Rückstand chromatographisch an Kieselgel mit dem Fließmittel Essigester gereinigt. Man erhielt 1,9 g des Produktes.
   ¹H-NMR (D₆-DMSO). δ = 4,3(2H), 6,3(1H), 7,35(1H), 7,5(1H), 7,7-7,9(5H), 8,3(2H), 9,4(1H) und 9,9(1H) ppm.

### Beispiel 25

### 2(4(3-Aminomethyl-pyrrol-1-yl)phenyl)-benzimidazol-4-carbonsäureamid

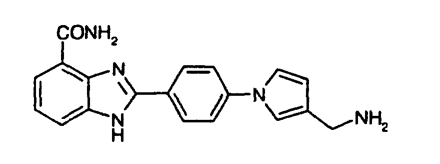

1,7 g (4 mMol) des Beispiels 24 wurden in 70 ml Tetrahydrofuran gelöst und mit einer Lösung aus 0,38 g (15,9 mMol) Lithiumhydroxid in 25 ml Wasser versetzt. Alles wurde für 2 Stunden bei Raumtemperatur gerührt. Anschließend wurde das Reaktionsgemisch mit verdünnter Salzsäure neutralisiert und das organische Lösungsmittel im Vakuum entfernt. Der anfallende Niederschlag wurde abgesaugt und getrocknet. Man erhielt 0,87 g des Produktes.
¹H-NMR (D₆-DMSO). δ = 4,4(2H), 7,0(NH) und 7,8-8,4(11H) ppm.

### Beispiel 26

### 2(4(3-Aminomethyl-pyrrol-1-yl)phenyl)-benzimidazol-4-carbonsäureamid x 2 Methan-sulfonsäure

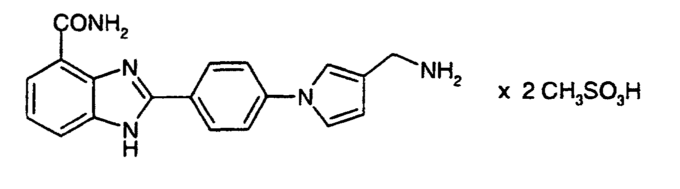

0,1 g des Produktes aus Beispiel 25 wurden in 2 ml Tetrahydrofuran gelöst und mit 20,5 µL Methansulfonsäure, verdünnt mit 5 ml Wasser, versetzt. Man verdünnte anschließend noch mit Wasser und lyophilisierte danach die so erhaltene Lösung, wobei man 117 mg des Produktes erhielt.
¹H-NMR (D₆-DMSO). δ = 2,45(6H), 4,0(2H), 6,4(1H), 7,2-8,4(11H) und 9,1(NH) ppm.

### Beispiel 27

### 2(4(1-Imidazolyl)phenyl)-benzimidazol-4-carbonsäureamid

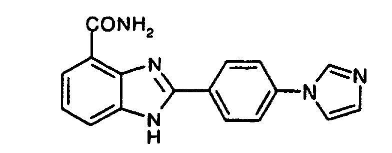

a) 2(4(1-Imidazolyl)phenyl)-benzimidazol-4-carbonsäureethylester
   1 g (5,5 mMol) 2,3-Diaminobenzoesäureethylester und 0,7 ml konzentrierte Essigsäure wurden in 13 ml Methanol gelöst. Anschließend wurden 1,24 g (7,2 mMol) 4-Imidazol-1-ylbenzaldehyd, gelöst in 25 ml Methanol, innerhalb von 30 Minuten zugetropft. Danach wurden 1,4 g (7,2 mMol) Kupfer-II-acetat, gelöst in 19 ml warmen Wasser, zügig zugetropft und anschließend alles für 20 Minuten unter Rückfluß gekocht. Man kühlte die Reaktionslösung auf 50°C und gab 2,25 ml 32%iger Salzsäure zu. Danach tropfte man noch vorsichtig eine Lösung aus 2,13 g Natriumsulfid-Hydrat in 15 ml Wasser zu und rührte alles noch für 15 Minuten. Die Reaktionslösung wurde auf Eiswasser gegossen und der anfallende Niederschag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 1,7 g des Zwischenproduktes.
b) 2(4(1-Imidazolyl)phenyl)-benzimidazol-4-carbonsäurehydrazid
   Zu 1,6 g (5,0 mMol) der Zwischenverbindung 27a in 30 ml Butanol wurden 5 ml Hydrazinhydrat gegeben und alles für 8 Stunden unter Rückfluß gekocht. Anschließend wurde das Reaktionsgemisch im Vakuum eingeengt und der Rückstand zwischen Wasser und Essigester verteilt. Die Essigester-Phase wurde abgetrennt, getrocknet und im Vakuum eingeengt. Man erhielt 0,55 g der Zwischenverbindung.
c) 2(4(1-Imidazolyl)phenyl)-benzimidazol-4-carbonsäureamid
   Zu 0,53 g (1,7 mMol) der Zwischenverbindung 27b in 35 ml Dimethylformamid/Wasser (2/1) wurden ca. 1,5 g Raney-Nickel gegeben und alles für 8 Stunden auf 100°C erwärmt. Anschließend wurde das Reaktionsgemisch filtriert und das Filtrat mit viel Wasser verdünnt, wobei das Produkt ausfiel. Man erhielt 0,19 g des Produktes.
   ¹H-NMR (D₆-DMSO). δ = 7,2(1H), 7,4(1H), 7,7-8,0(6H) 8,4(3H) und 9,4(1H) ppm.

### Beispiel 28

### 2(4(1-Imidazolyl)phenyl)-benzimidazol-4-carbonsäureamid x 2Methansulfonsäure

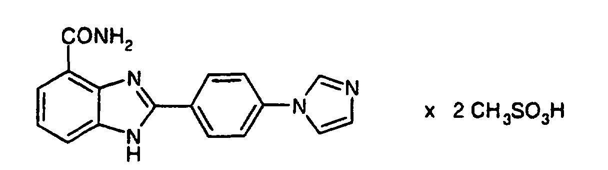

50 mg des Beispiels 4 wurden analog der Vorschrift 26a in das Bismethansulfonat überführt und lyophilisiert. Man erhielt 60 mg des Produktes.
¹H-NMR (D₆-DMSO). δ = 2,3(6H), 7,4(2H), 7,8-8,2(7H), 8,4(1H), 8,5(2H), 9,1(1H) und 9,8 (2H) ppm.

### Beispiel 29

### 2(3(3-Trifluoracetamidomethyl-pyrrol-1-yl)phenyl)-benzimidazol-4-carbonsäureamid

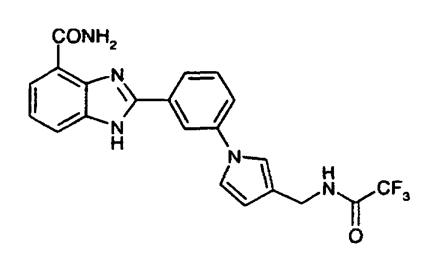

a) 2(3-Nitrophenyl)-benzimidazol-4-carbonsäureethylester
   4,2 g (23 mMol) 2,3-Diaminobenzoesäureethylester und 3,1 ml konzentrierte Essigsäure wurden in 100 ml Methanol gelöst. Anschließend wurden 4,5 g (30 mMol) 4-Nitrobenzaldehyd, gelöst in 150 ml Methanol, innerhalb von 30Minuten zugetropft. Danach wurden 6 g (30 mMol) Kupfer-II-acetat, gelöst in 150 ml warmen Wasser, zügig zugetropft und anschließend alles für 20 Minuten unter Rückfluß gekocht. Man kühlte die Reaktionslösung auf 50°C und gab 8,3 ml konzentrierte Salzsäure zu. Danach tropfte man noch vorsichtig eine Lösung aus 8,6 g Natriumsulfid-Hydrat in 100 ml Wasser zu und rührte alles noch für 15 Minuten. Die Reaktionslösung wurde auf Eiswasser gegossen und der anfallende Niederschlag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 6.1g des Zwischenproduktes.
b) 2(3-Nitro-phenyl)-benzimidazol-4-carbonsäurehydrazid
   Zu 6 g (19,3 mMol) der Zwischenverbindung 29a in 70 ml Ethanol wurden 4,8 g (96 mMol) Hydrazinhydrat gegeben und alles für 3 Stunden unter Rückfluß gekocht. Anschließend wurde der Reaktionsansatz auf Wasser gegossen und der entstandene Niederschlag abgesaugt. Man erhielt 4,8 g der Zwischenverbindung.
c) 2(3-Amino-phenyl)-benzimidazol-4-carbonsäureamid
   Zu 4,7 g (15,8 mMol) der Zwischenverbindung 29b in 400 ml Ethanol wurden 0,5 g Palladium-Kohle (10%ig) gegeben und alles mit Wasserstoff hydriert. Anschließend wurde der Reaktionsansatz filtriert und im Vakuum eingeengt. Der Rückstand wurde in 100 ml Dimethylformamid aufgenommen und danach mit 70 ml Wasser verdünnt. Anschließend wurden 10 g Raney-Nickel zugegeben und alles für 2 h auf 90°C erwärmt. Danach wurde filtriert und das Filtrat im Vakuum eingeengt. Der erhaltene Rückstand wurde aus Essigester/Ether kristallisiert, wonach 3,1 g des Produktes erhalten wurden.
d) 2(3(3-Triflouracetamidomethyl-pyrrol-1-yl)phenyl)-benzimidazol-4-carbonsäureamid
   2,2 g (8,7 mMol) der Zwischenverbindung 29c und 2,8 g (10,9 mMol) 2,5-Dimethoxy-3-(trifluoracet-amidomethyl)tetrahydrofuran wurden in 75 ml konzentrierter Essigsäure gegeben und für 15 Minuten unter Rückfluß erwärmt. Anschließend wurde alles im Vakuum eingeengt und der anfallende Rückstand chromatographisch an Kieselgel mit dem Fließmittel Essigester/Methanol (10/1) gereinigt. Man erhielt 2,5 g des Produktes.
   MS: m/e = 427 (M⁺).

### Beispiel 30

### 2(3(3-Aminomethyl-pyrrol-1-yl)phenyl)-benzimidazol-4-carbonsäureamid

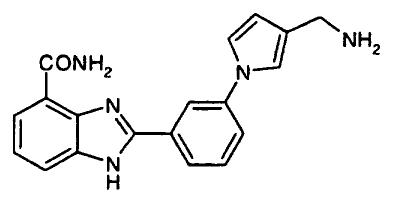

2,3 g (5,4 mMol) des Beispiels 29 wurden in 100 ml Tetrahydrofuran gelöst und mit 0,26 g (10,8 mMoll) Lithiumhydroxid, gelöst in 50 ml Wasser, versetzt. Alles wurde für 2 Stunden bei Raumtemperatur gerührt. Anschließend wurde der Ansatz durch Zugabe von verdünnter Salzsäure neutralisiert und das organische Lösungsmittel im Vakuum entfernt. Der langsam auskristallisierende Niederschlag wurde abgesaugt. Man erhielt 0,61 g des Produktes.
¹H-NMR (CF₃COOD). δ = 4,4(2H), 7,0(NH) und 7,8-8,4(11H) ppm.

### Beispiel 31

### 2(4(4-Methylpiperazin-1-yl)phenyl)-benzimidazol-4-carbonsäureamid

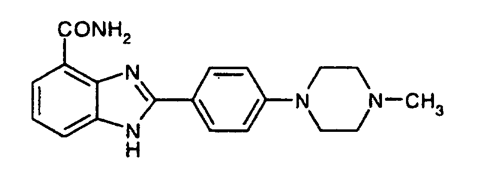

a) 4(4-Methylpiperazin-1-yl)benzaldehyd
   20 g (161 mMol) 4-Fluorbenzaldehyd, 48,4 g (483 mMol) 1-Methylpiperazin und 22,3 g (161 mMol) Kaliumkarbonat wurden in 50 ml Dimethylformamid gegeben und für 36 Stunden auf 130°C erwärmt. Anschließend wurde der Ansatz im Vakuum eingeengt. Der Rückstand wurde zwischen Essigester und 2 M Salzsäure verteilt. Die wäßrige Phase wurde abgetrennt und mit wäßriger Natriumhydrogenkarbonat-Lösung alkalisiert. Diese wäßrige Phase wurde mit Essigester extrahiert, die organische Phase abgetrennt, getrocknet und im Vakuum eingeengt. Man erhielt 48,7 g des Zwischenproduktes.
b) 2(4(4-Methylpiperazin-1-yl)phenyl)-benzimidazol-4-carbonsäureethylester
   1,5 g (8,3 mMol) 2,3-Diaminobenzoesäureethylester und 2,2 g (10,8 mMol) der Zwischenverbindung 8a wurden analog der Vorschrift 6a umgesetzt, wobei man nach einer chromatographischen Reinigung an Kieselgel 2,8 g des Produktes erhielt.
c) 2(4(4-Methylpiperazin-1-yl)phenyl)-benzimidazol-4-carbonsäurehydrazid
   1,35 g (3,7 mMol) der Zwischenverbindung 21b wurden analog der Vorschrift 6b mit Hydrazin umgesetzt, wobei 1,1 g des Produktes anfielen.
d) 2(4(4-Methylpiperazin-1-yl)phenyl)-benzimidazol-4-carbonsäureamid
   Die Zwischenverbindung wurde analog der Vorschrift 29c mit Raney-Nickel behandelt, wonach das Produkt erhalten wurde.
   ¹H-NMR (D₆-DMSO). δ = 2,25(3H), 2,6(4H), 3,2(4H), 7,0-8,1(9H) und 9,5(1H) ppm.

### Beispiel 32

### 2(3(2-Trifluoracetamidomethyl-pyrrol-1-yl)phenyl)-benzimidazol-4-carbonsäureamid

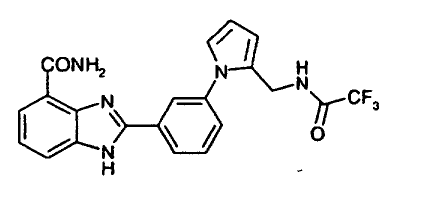

Analog Beispiel 29 wurde aus 2,3-Diaminobenzoesäureethylester, 3-Nitrobenzaldehyd und 2,5-Dimethoxy-2-(trifluoracetamidomethyl)-tetrahydrofuran die obige Verbindung hergestellt.
¹H-NMR (D₆-DMSO). δ = 4,5(2H), 6,3(2H), 7,3-8,0(6H), 9,25(1H) und 9,8(1H) ppm.

### Beispiel 33

### 2(3(3-Formyl-pyrrol-1-yl)phenyl)-benzimidazol-4-carbonsäureamid

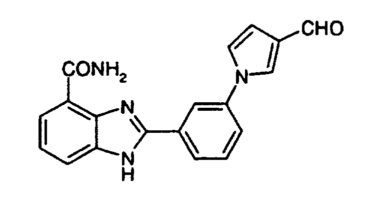

Analog Beispiel 29 wurde aus 2,3-Diaminobenzoesäureethylester, 3-Nitrobenzaldehyd und 2,5-Dimethoxy-tetrahydrofuranyl-3-carbaldehyd die obige Verbindung hergestellt.
¹H-NMR (D₆-DMSO). δ = 6,8(2H), 7,3-8,0(6H), 8,3(1H), 8,4(1H), 8,6(1H), 9,2(1H) und 9,8(1H) ppm.

### Beispiel 34

### 2(3(3(N,N-Benzyl-methylaminomethyl)-pyrrol-1-yl)phenyl)-benzimidazol-4-carbonsäureamid x 2 HCl

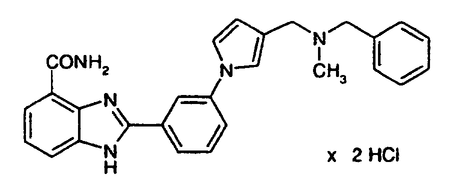

2,0 g (6,0 mMol) der Verbindung aus Beispiel 33, 0,74 g (6,0 mMol) N-Methylbenzylamin, und 0,4 ml (6,0 mMol) Eisessig wurden in 100 ml Ethanol gelöst. Bei Raumtemperatur wurden danach portionsweise 0,38 g (6,0 mMol) Natriumcyanoborhydrid zugefügt und alles bei Raumtemperatur für 16 h gerührt. Anschließend wurde der Ansatz mit wäßriger Natriumhydrogenkarbonat-Lösung verdünnt und mit Essigester extrahiert. Die organische Phase wurde abgetrennt, getrocknet und im Vakuum eingeengt. Der Rückstand wurde chromatographisch an Kieselgel (Fließmittel: Essigester/Methanol = 10/1) gereinigt. Das so erhaltene Produkt wurde in Aceton gelöst und mit isopropanolischer Chlorwasserstoff-Lösung versetzt, wobei das Produkt ausfiel und abgesaugt wurde. Man erhielt 0,98 g des Produktes.
¹H-NMR (D₆-DMSO). δ = 2,6(3H), 4,1-4,5(4H), 6,6(1H), 7,3-8,0(13H), 8,2(1H), 8,6(1H), 9,1(1H) und 10,8(1H) ppm.

### Beispiel 35

### 2(3(2-Aminomethyl-pyrrol-1-yl)phenyl)-benzimidazol-4-carbonsäureamid

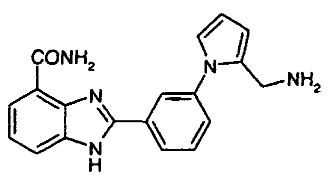

1,0 g (2,3 mMol) der Verbindung aus Beispiel 32 wurden in 100 ml Wasser gelöst und mit 0,56 g (23,4 mMol) Lithiumhydroxid, gelöst in 20 ml Wasser, versetzt. Alles wurde für 90 Minuten bei Raumtemperatur gerührt. Anschließend wurde das organische Lösungsmittel im Vakuum entfernt und die resultierende wäßrige Phase mit verdünnter Salzsäure vorsichtig neutralisiert. Der auftetende Niederschlag wurde abgesaugt. Man erhielt 0,55 g des Produktes.
¹H-NMR (D₆-DMSO). δ = 3,8(2H), 6,2(2H), 7,0(1H), 7,35(1H), 7,6-8,1(5H), 8,3(1H), 9,35(1H) und 9,5(1H) ppm.

### Beispiel 36

### 2(4(4-Methylpiperazin-1-yl)phenyl)-benzimidazol-4-carbonsäureamid x 3 HCl

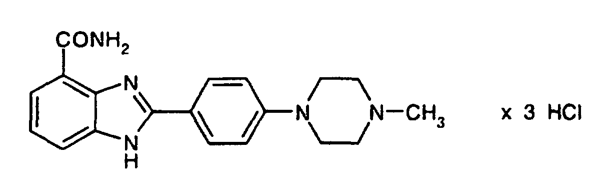

0,25 g des Produktes aus Beispiel 31 wurden in 25 ml Essigester/Tetrahydrofuran (4/1) gelöst und tropfenweise mit etherischer Chlorwasserstoffsäure versetzt. Der entstandene Niederschlag wurde mit Aceton behandelt und abgesaugt. Man erhielt 0,25 g des Produktes.
¹H-NMR (D₆-DMSO). δ = 2,75(3H), 3,1-3,4(4H), 4,0-4,4(4H), 7,25(2H), 7,5(1H), 7,9-8,1(4H), 8,3(2H), 9,0(breit) und 11,5(breit) ppm.

### Beispiel 37

### 2(4(4-tert-Butyloxypiperazin-1-yl)phenyl)-benzimidazol-4-carbonsäureamid

¹H-NMR (D₆-DMSO). δ = 1,4(9H), 3,3(4H), 3,5(4H), 7,2(1H), 7,3(1H), 7,7(1H), 7,75(1H), 7,8(1H), 8,2(2H), 9,4(1H) und 12,5 ppm.

### Beispiel 38

### 2(4(Piperazin-1-yl)phenyl)-benzimidazol-4-carbonsäureamid x 2 HCl

¹H-NMR (D₆-DMSO). δ = 3,3(4H), ca. 3,7(4H), 7,3(2H), 7,6(1H), 7,9-8,0(3H), 8,3(2H), 8.7(1H) und 9,5(breit) ppm.

### Beispiel 39

### 2(3(2(Aminomethyl)-pyrrol-1-yl)phenyl)-benzimidazol-4-carbonsäureamid x 2 HCl

¹H-NMR (D₂O). δ = 4,25(2H), 6,4(1H), 6,6(1H), 7,1(1H), 7,4(1H), 7,6(1H), 7,7-7,8(3H), 7,9(1H) und 8,0(1H) ppm.

### Beispiel 40

### 2(4(3-Formylpyrrol-1-yl)phenyl)-benzimidazol-4-carbonsäureamid

¹H-NMR (D₆-DMSO). δ = 6,7(1H), 7,3(1H), 7,7-8,0(7H), 8,4(2H), 9,4(1H), 9,8(1H) und 13,5(breit) ppm.

### Beispiel 41

### 2(4(3-(N,N-Benzylmethylaminomethyl)-pyrrol-1-yl)phenyl)-benzimidazol-4-carbonsäureamid x 2 HCl

MS: m/e = 435 (M⁺).

### Beispiel 42

### 2(4(3-(N,N-Diethylaminomethyl)-pyrrol-1-yl)phenyl)-benzimidazol-4-carbonsäureamid x 2 HCl

¹H-NMR (D₆-DMSO). δ = 1,3(6H), 3,1(4H), 4,2(2H), 6,6(1H), 7,5(1H), 7,75(1H), 7,8-8,0(6H), 8,5(2H), 9,1(1H) und 10,4(1H) ppm.

### Beispiel 43

### 2(4(3-(4-Methylpiperazin-1-ylmethyl)-pyrrol-1-yl)phenyl)-benzimidazol-4-carbonsäureamid

¹H-NMR (D₆-DMSO). δ = 2,1(3H), 2,2-2,5(8H), 3,35(2H), 6,2(1H), 7,3-8,0(7H), 8,3(2H) und 9,4(breit) ppm.

### Beispiel 44

### 2(4(3-(4-Benzylpiperazin-1-ylmethyl)-pyrrol-1-yl)phenyl)-benzimidazol-4-carbonsäureamid

¹H-NMR (D₆-DMSO). δ = 2,2-2,6(8H), 3,4(2H), 3,5(2H), 6,2(1H), 7,2-8,0(13H), 8,3(2H), 9,4(1H) und 13,4(breit) ppm.

### Beispiel 45

### 2(4(3-(Piperidin-1-ylmethyl)-pyrrol-1-yl)phenyl)-benzimidazol-4-carbonsäureamid

¹H-NMR (D₆-DMSO). δ = 1,3-1,6(6H), 2,3(4H), 3,3(2H), 6,2(1H), 7,3-8,0(8H), 8,3(2H) und 9,4(breit) ppm.

### Beispiel 46

### 2(4(4-Benzylpiperazin-1-yl)phenyl)-benzimidazol-4-carbonsäureamid 3 x HCl

¹H-NMR (D₆-DMSO). δ = 3,2(4H), 4,2(4H), 4,5(2H), 7,2(2H), 7,4-8,0(9H), 8,2(2H), 9,0(1H) und 11,8(breit) ppm.

### Beispiel 47

### 2(4(4-Cyclohexylpiperazin-1-yl)phenyl)-benzimidazol-4-carbonsäureamid

¹H-NMR (D₆-DMSO). δ = 1,1-1,9(10H), 2,7(4H), 3,2(4H), 4,1(1H), 7,1(2H), 7,25(1H), 7,7(2H), 7,8(1H), 8,0(2H), 9,4(1H) und ca. 13(breit) ppm.

### Beispiel 48

### 2(4(4-Ethylpiperazin-1-yl)phenyl)-benzimidazol-4-carbonsäureamid

¹H-NMR (D₆-DMSO). δ = 1,0(3H), 2,4(2H), 2,5(4H), 3,2(4H), 7,0-7,3(3H), 7,6-7,9(2H), 8,0(2H), 9,4(1H) und ca. 13(breit) ppm.

### Beispiel 49

### 2(4(4-n-Butylpiperazin-1-yl)phenyl)-benzimidazol-4-carbonsäureamid

¹H-NMR (D₆-DMSO). δ = 0,9(3H), 1,2-1,6(4H), 2,3(2H), 3,2-3,5(8H), 7,1(2H), 7,3(1H), 7,6-7,9(3H), 8,1(2H), 9,5(1H) und 13(breit)ppm.

### Beispiel 50

### 2(4(4-Diphenylmethylpiperazin-1-yl)phenyl)-benzimidazol-4-carbonsäureamid

¹H-NMR (D₆-DMSO). δ = 2,5(4H), 3,2(4H), 4,3(1H), 7,0-7,9(16H), 8,1(2H), 9,4(1H) und ca. 13(breit) ppm.

### Beispiel 51

### 2(2-Methyl-4-piperazin-1-yl-phenyl)-benzimidazol-4-carbonsäureamid 3 x HCl

MS: m/e = 335(M⁺).

### Beispiel 52

### 2(3-Piperazin-1-yl-phenyl)-benzimidazol-4-carbonsäureamid 3 x HCl

¹H-NMR (D₆-DMSO). δ = 3,2(4H), 3,6(2H), 7,2-7,6(3H), 7,7-8,0(4H), 8,9(breit) und 9,5(breit) ppm.

### Beispiel 53

### 2(4(4-Isopropylpiperazin-1-yl)phenyl)-benzimidazol-4-carbonsäureamid

¹H-NMR (D₆-DMSO). δ = 1,0(6H), 2,7(4H), 2,8(1H), 3,3(4H), 7,1(2H), 7,2(1H), 7,5-7,9(3H), 8,05(2H), 9,4(1H) und 13(breit) ppm.

### Beispiel 54

### 2(4(4-tert.Butyloxycarbonylhomopiperazin-1-yl)phenyl)-benzimidazol-4-carbonsäureamid

¹H-NMR (D₆-DMSO). δ = 1,1-1,3(9H), 1,9(2H), 3,1-3,9(8H), 6,9(2H), 7,2(1H), 7,7-7,9(3H), 8,0(2H), 9,5(1H) und ca. 13(breit) ppm.

### Beispiel 55

### 2(4(Homopiperazin-1-yl)phenyl)-benzimidazol-4-carbonsäureamid

¹H-NMR (D₆-DMSO). δ = 2,1(2H), 3,1(2H), 3,2(2H), 3,7(2H), 3,9(2H), 7,0(2H), 7,5(1H), 7,8-8,0(3H), 8,2(2H), 8,7(breit) und 9,3(breit) ppm.

### Beispiel 56

### 2(4(4-(Piperidin-1-yl)piperidin-1-yl)phenyl)-benzimidazol-4-carbonsäureamid

¹H-NMR (D₆-DMSO). δ = 1,7-1,9(8H), 2,2(2H), 2,8-2,9(3H), 3,3(4H), 4,1(2H), 7,1(2H), 7,3(1H), 7,7(1H), 7,75(1H), 7,8(1H), 8,1(2H), 9,4(1H) und 13,2(breit) ppm.

### Beispiel 57

### 2(4(3-Amino-pyrroldin-1-yl)phenyl)-benzimidazol-4-carbonsäureamid x 2 HCl

MS: m/e = 321 (M⁺).

### Beispiel 58

### 2(4(4-Benzyl-homopiperazin-1-yl)phenyl)-benzimidazol-4-carbonsäureamid

### Beispiel 59

### 2(4(4-Methyl-homopiperazin-1-yl)phenyl)-benzimidazol-4-carbonsäureamid

### Beispiel 60

### 2(4(4-Ethy1-homopiperazin-1-yl)phenyl)-benzimidazol-4-carbonsäureamid

### Beispiel 61

### 2(4(4-Isopropyl-homopiperazin-1-yl)phenyl)-benzimidazol-4-carbonsäureamid

### Beispiel 62

### 2(4(4-Butyl-homopiperazin-1-yl)phenyl)-benzimidazol-4-carbonsäureamid

### Beispiel 63

### Synthese von 2-Phenylbenzimidazol-4-carboxamid

a) 2,3-Diamino-benzoesäureamid x 2 Hydrochlorid
   Eine Lösung von 200 g (1,11 mol) 2,3-Diaminobenzoesäureethylester in 1500 ml 1-Butanol wurde bei Raumtemperatur vorsichtig mit 400 ml Hydrazinhydrat versetzt. Die Mischung wurde 15 Stunden auf 100°C erwärmt. Anschließend wurde der Ansatz auf ein Drittel des Volumens eingeengt. Diese Lösung wurde langsam zu einer Suspension von ca. 200 g Raney-Nickel in 500 ml Wasser und 1000 ml Dimethylformamid getropft. Die Mischung wurde 2 Stunden auf 100°C erwärmt. Nach Abkühlen auf 10°C wurde der Katalysator abgetrennt und das Filtrat im Vakuum eingeengt. Das so erhaltene Öl wurde in 500 ml Methanol gelöst und mit Diethylether versetzt. Der Niederschlag wurde abgetrennt und das Filtrat erneut eingeengt. Eine Lösung des erhaltenen Öls in Methanol wurde unter Rückfluß mit Chlorwasserstoff/iso-Propanol versetzt. Der beim Abkühlen ausfallende Niederschlag wurde abgesaugt, mit Diethylether aufgeschlämmt und erneut abgesaugt. Man erhielt 172,2 g des Produktes.
b) 2-Phenylbenzimidazol-4-carboxamid
   Zu einer Lösung von 0,84 g (15 mmol) Kaliumhydroxidpulver in 100 ml Ethanol wurden bei Raumtemperatur 1,68 g (7,5 mmol) des Produktes aus 1b zugegeben. Nach 5 Minuten wurden 1,35 g (22,5 mmol) Eisessig zugegeben, und innerhalb von 30 Minuten wurde eine Lösung von 1 g (9,38 mmol) Benzaldehyd in 20 ml Ethanol zugetropft. Anschließend wurde eine Lösung von 2,59 g (12,97 mmol) Kupfer-II-acetat in 20 ml dest. Wasser zügig zugetropft. Die Mischung wurde 2 Stunden unter Rückfluß erhitzt. Der Ansatz wurde auf Wasser gegeben, mit Konzentrierter Ammoniaklösung alkalisch gestellt und mit Ethylacetat extrahiert. Die organische Phase wurde mit Wasser gewaschen und unter Zusatz von Aktivkohle über Magnesiumsulfat getrocknet und im Vakuum eingeengt. Der harzige Rückstand wurde mit Diethylether verrieben, das abgetrennte Kristallisat wurde mit Diethylether gewaschen und im Vakuum getrocknet. Es wurden 1,5 g des Produktes erhalten.

## Patentansprüche

1. Verbindungen der Formel I oder 11 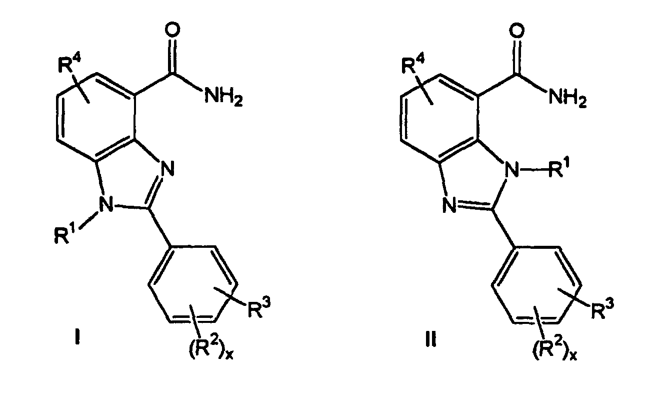 worin
R¹ Wasserstoff, verzweigtes und unverzweigtes C₁-C₆-Alkyl, wobei ein C-Atom des Alkyl-Restes noch OR¹¹ oder eine Gruppe R⁵ tragen kann, wobei R¹¹ Wasserstoff oder C₁-C₄-Alkyl bedeutet, und
R² Wasserstoff, Chlor, Brom, Jod, Fluor, CF₃, Nitro, NHCOR²¹, NR²²R²³, OH, O-C₁-C₄-Alkyl, O-C₁-C₄-Alkyl-Phenyl, NH₂, Phenyl, wobei die Phenyl-Ringe noch mit maximal zwei Resten R²⁴ substituiert sein können, und R²¹ und R²² unabhängig voneinander Wasserstoff oder C₁-C₄-Alkyl bedeuten und R²³ Wasserstoff, C₁-C₄-Alkyl oder Phenyl bedeuten, und R²⁴ OH, C₁-C₆-Alkyl, O-C₁-C₄-Alkyl, Chlor, Brom, Jod, Fluor, CF₃, Nitro, NH₂, und
x 0, 1 und 2 sein kann und
R³ -D-(F¹)ₚ-(E)_{q}-(F²)ᵣ-G bedeutet, wobei p, q und r nicht gleichzeitig 0 sein können, oder -E- (D)ᵤ-(F²)ₛ-(G)ᵥ, wobei der Rest E noch mit einem oder zwei Resten A substituiert sein kann, und
R⁴ Wasserstoff, Chlor, Fluor, Brom, Jod, verzweigtes und unverzweigtes C₁-C₆-Alkyl, OH, Nitro, CF₃, CN, NR⁴¹R⁴², NH-CO-R⁴³, O-C₁-C₄-Alkyl, wobei R⁴¹ und R⁴² unabhängig voneinander Wasserstoff oder C₁-C₄-Alkyl bedeuten und
R⁴³ Wasserstoff, C₁-C₄-Alkyl, C₁-C₄-Alkyl-Phenyl oder Phenyl bedeuten, und
D S und O
E Phenyl, Imidazol, Pyrrol, Thiophen, Pyridin, Pyrimidin, Piperazin, Pyrazin, Furan, Thiazol, Isoxazol, Pyrrolidin, Piperidin, Trihydroazepin und
F¹ eine Kette aus 1 bis 8 Kohlenstoffatomen, wobei ein Kohlenstoffatom der Kette noch eine OH oder O-C₁-C₄-Alkyl-Gruppe tragen kann und
F² eine Kette aus 1 bis 8 Kohlenstoffatomen, wobei ein Kohlenstoffatom der Kette noch eine OH oder O-C₁-C₄-Alkyl-Gruppe tragen kann und
p 0 und 1 bedeuten kann und
q 0 und 1 sein kann, und
r 0 und 1 sein kann und
s 0 und 1 sein kann und
u 0 und 1 sein kann und
v 0 und 1 sein kann
G NR⁵¹R⁵² und 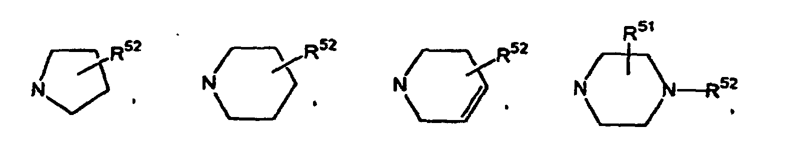 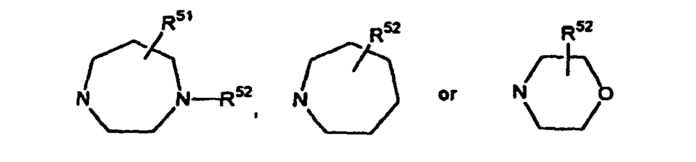 sein kann und
R⁵¹ Wasserstoff und verzweigtes und unverzweigtes C₁-C₆-Alkyl, (CH₂)ₜ-K bedeutet und
R⁵² Wasserstoff, verzweigtes und unverzweigtes C₁-C₆-Alkyl, Phenyl, 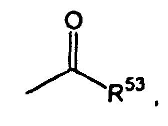 -SO₂R⁵³, -(C=N)R⁵³, -(C=N)-NHR⁵³ oder -CO-NHR⁵³
worin
R⁵³ verzweigtes oder unverzweigtes O-C₁-C₆-Alkyl, Phenyl, verzweigtes oder unverzweigtes C₁-C₄-Alkyl-Phenyl, wobei bei R⁵² und R⁵³ unabhängig voneinander ein Wasserstoff des C₁-C₆-Alkylrests durch einen der folgenden Reste substituiert sein kann: OH, O-C₁-C₄-Alkyl, Cyclohexyl, Cyclopentyl, Tetrahydronaphthyl, Cyclopropyl, Cyclobutyl, Cycloheptyl, Naphthyl und Phenyl, wobei die Carbocyclen der Reste R⁵² und R⁵³ unabhängig voneinander noch einen oder zwei der folgenden Reste tragen können: verzweigtes oder unverzweigtes C₁-C₆-Alkyl, verzweigtes oder unverzweigtes O-C₁-C₄-Alkyl, OH, F, Cl, Br, I, CF₃, NO₂, NH₂, CN, COOH, COOC₁-C₄-Alkyl, C₁-C₄-Alkyl-amino, CCl₃, C₁-C₄-Dialkylamino, SO₂-C₁-C₄-Alkyl, SO₂Phenyl, CONH₂, CONH-C₁-C₄-Alkyl, CONHPhenyl, CONH-C₁-C₄-Alkyl-Phenyl, NHSO₂-C₁-C₄-Alkyl, NHSO₂Phenyl, S-C₁-C₄-Alkyl, 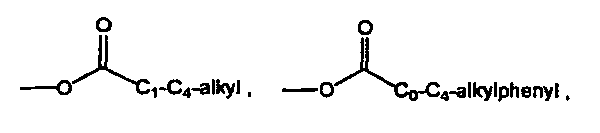 CHO, CH₂-O-C₁-C₄-Alkyl, -CH₂O-C₁-C₄-Alkyl-Phenyl, -CH₂OH, -SO-C₁-C₄-Alkyl, -SO-C₁-C₄-Alkyl-Phenyl, -SO₂NH₂, -SO₂NH-C₁-C₄-Alkyl
und zwei Reste eine Brücke -O-(CH₂)_{1,2}-O- bilden,
bedeuten kann,
A Wasserstoff, Chlor, Brom, Jod, Fluor, CF₃, Nitro, OH, O-C₁-C₄-Alkyl, O-C₁-C₄-Alkyl-Phenyl, NH₂, verzweigtes und unverzweigtes C₁-C₆-Alkyl, CN, NH-CO-R³³, wobei R³³ Wasserstoff, C₁-C₄-Alkyl oder Phenyl bedeutet, sein kann und
t 0, 1, 2, 3, 4 und
K Phenyl, der noch maximal zwei Reste R tragen kann, NR^{k1}R^{k2} (mit R^{k1} bzw. R^{k2} mit den gleichen Bedeutungen wie R⁴¹ bzw. R⁴²), NH-C₁-C₄-Alkyl-Phenyl, Pyrrolidin, Piperidin, 1,2,5,6-Tetrahydropyridin, Morpholin, Trihydroazepin, Piperazin, das noch mit einem Alkyl-Rest C₁-C₆-Alkyl substituiert sein kann, und Homopiperazin, das noch mit einem Alkyl-Rest C₁-C₆-Alkyl substituiert sein kann, und
R⁵ Wasserstoff, C₁-C₆-Alkyl, NR⁷R⁹ und 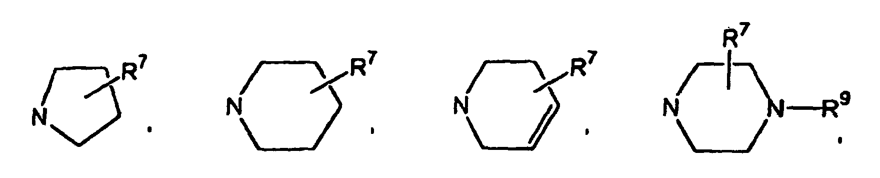 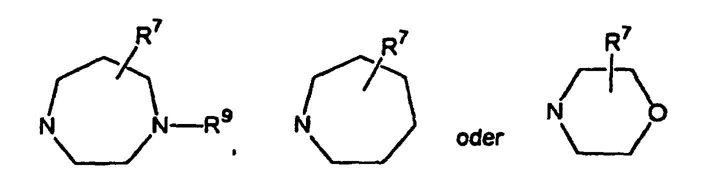 bedeuten kann und
R⁷ Wasserstoff, C₁-C₆-Alkyl, C₁-C₄-Alkyl-Phenyl, Phenyl,
wobei die Ringe noch mit bis zu zwei Resten R⁷¹ substituiert sein können, und
R⁷¹ OH, C₁-C₆-Alkyl, O-C₁-C₄-Alkyl, Chlor, Brom, Jod, Fluor, CF₃, Nitro, NH₂,
R⁸ Wasserstoff, C₁-C₆-Alkyl, Phenyl, C₁-C₄-Alkyl-Phenyl, wobei der Ring noch mit bis zu zwei Resten R⁸¹ substituiert sein kann, und
R⁸¹ OH, C₁-C₆-Alkyl, O-C₁-C₄-Alkyl, Chlor, Brom, Jod, Fluor, CF₃, Nitro, NH₂, und
R⁹ Wasserstoff, COCH₃, CO-O-C₁-C₄-Alkyl, COCF₃, verzweigtes und unverzweigtes C₁-C₆-Alkyl, wobei ein oder zwei Wasserstoffe des C₁-C₆-Alkylrests durch jeweils einen der folgenden Reste substituiert sein kann: OH, O-C₁-C₄-Alkyl und Phenyl und der Phenyl-Ring noch einen oder zwei der folgenden Reste tragen kann: Jod, Chlor, Brom, Fluor, verzweigtes und unverzweigtes C₁-C₆-Alkyl, Nitro, Amino, C₁-C₄-Alkylamino, C₁-C₄-Dialkylamino, OH, O-C₁-C₄-Alkyl, CN, CF₃, SO₂-C₁-C₄-Alkyl, bedeuten kann, und
sowie ihre tautomeren Formen, möglichen enantiomeren und diastereomeren Formen, und deren Phosphate, Carbamate von Aminosäuren, Ester und pharmakologisch verträglichen Salze.

2. Verbindungen der allgemeinen Formel I oder II nach Anspruch 1, worin
R¹ Wasserstoff, verzweigtes und unverzweigtes C₁-C₆-Alkyl, wobei ein C-Atom des Alkyl-Restes noch OR¹¹ oder eine Gruppe R⁵ tragen kann, wobei
R¹¹ Wasserstoff oder C₁-C₄-Alkyl bedeutet, und
R² Wasserstoff, Chlor, Fluor, Brom, Jod, verzweigtes und unverzweigtes C₁-C₆-Alkyl, Nitro, CF₃, CN, NR²¹R²², NH-CO-R²³, OR²¹, wobei
R²¹ und R²² unabhängig voneinander Wasserstoff oder C₁-C₄-Alkyl bedeuten und
R²³ Wasserstoff, C₁-C₄-Alkyl oder Phenyl bedeuten, und
R³ -O-(CH₂)ₒ-(CHR³¹)ₘ-(CH₂)ₙ-R⁵, wobei
R³¹ Wasserstoff, C₁-C₄-Alkyl, OH und O-C₁-C₄-Alkyl,
m, o unabhängig voneinander 0, 1 oder 2 bedeutet, und
n 1, 2, 3 oder 4 bedeutet, und
R⁴ Wasserstoff, verzweigtes und unverzweigtes C₁-C₆-Alkyl, Chlor, Brom Fluor, Nitro, Cyano, NR⁴¹R⁴², NH-CO-R⁴³, OR⁴¹, wobei
R⁴¹ und R⁴² unabhängig voneinander Wasserstoff oder C₁-C₄-Alkyl bedeuten und
R⁴³ C₁-C₄ Alkyl oder Phenyl bedeuten, und
R⁵ NR⁵¹R⁵² oder einen der folgenden Reste 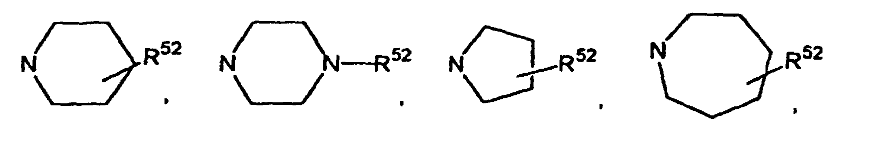 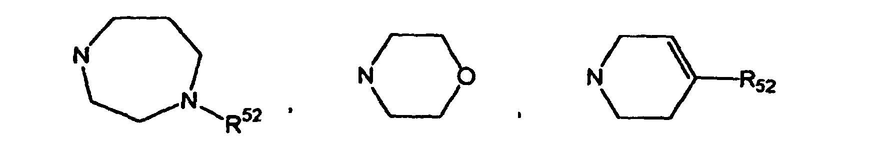
bedeutet, wobei
R⁵¹ Wasserstoff und verzweigtes und unverzweigtes C₁-C₆-Alkyl bedeutet und
R⁵² Wasserstoff, verzweigtes und unverzweigtes C₁-C₆-Alkyl, Phenyl, 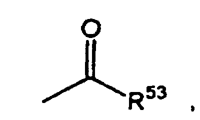 -SO₂R⁵³, worin
R⁵³ verzweigtes oder unverzweigtes O-C₁-C₆-Alkyl, Phenyl, verzweigtes oder unverzweigtes C₁-C₄-Alkyl-Phenyl, wobei bei R⁵² und R⁵³ unabhängig voneinander ein Wasserstoff des C₁-C₆-Alkylrests durch einen der folgenden Reste substituiert sein kann: OH, O-C₁-C₄-Alkyl, Cyclohexyl, Cyclopentyl, Tetrahydronaphthyl, Cyclopropyl, Cyclobutyl, Cycloheptyl, Naphthyl und Phenyl, wobei die Carbocyclen der Reste R⁵² und R⁵³ unabhängig voneinander noch einen oder zwei der folgenden Reste tragen können: verzweigtes oder unverzweigtes C₁-C₆-Alkyl, verzweigtes oder unverzweigtes O-C₁-C₄-Alkyl, OH, F, Cl, Br, I, CF₃, NO₂, NH₂, CN, COOH, COOC₁-C₄-Alkyl, C₁-C₄-Alkyl-amino, CCl₃, C₁-C₄-Dialkylamino, SO₂-C₁-C₄-Alkyl, SO₂Phenyl, CONH₂, CONH-C₁-C₄-Alkyl, CONHPhenyl, CONH-C₁-C₄-Alkyl-Phenyl, NHSO₂-C₁-C₄-Alkyl, NHSO₂Phenyl, S-C₁-C₄-Alkyl, 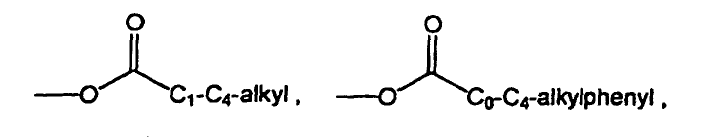 CHO, CH₂-O-C₁-C₄-Alkyl, -CH₂O-C₁-C₄-Alkyl-Phenyl, -CH₂OH, -SO-C₁-C₄-Alkyl, -SO-C₁-C₄-Alkyl-Phenyl, -SO₂NH₂, -SO₂NH-C₁-C₄-Alkyl
und zwei Reste eine Brücke -O-(CH₂)_{1,2}-O- bilden,
bedeuten kann,
sowie ihre tautomeren Formen, möglichen enantiomeren und diastereomeren Formen, deren Phosphate, Carbamate von Aminosäuren, Ester sowie mögliche physiologisch verträgliche Salze.

3. Verbindungen der allgemeinen Formel I oder II nach Anspruch 1, worin
R¹ Wasserstoff, verzweigtes und unverzweigtes C₁-C₆-Alkyl, wobei ein C-Atom des Alkyl-Restes noch OR¹¹ oder eine Gruppe R⁵ tragen kann, wobei
R¹¹ Wasserstoff oder C₁-C₄-Alkyl bedeutet, und
R² Wasserstoff, Chlor, Fluor, Brom, Jod, verzweigtes und unverzweigtes C₁-C₆-Alkyl, Nitro, CF₃, CN, NR²¹R²², NH-CO-R²³, OR²¹, wobei
R²¹ und R²² unabhängig voneinander Wasserstoff oder C₁-C₄-Alkyl bedeuten und
R²³ Wasserstoff, C₁-C₄-Alkyl oder Phenyl bedeuten, und
R³ 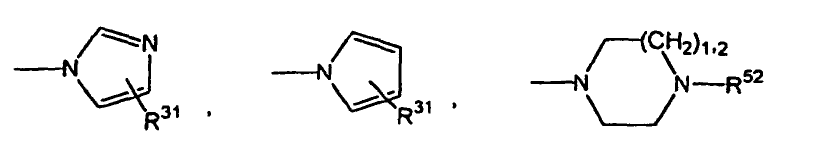 und
R³¹ Wasserstoff, CHO und -(CH₂)ₒ-(CHR³²)ₘ-(CH₂)ₙ-R⁵ wobei R³² Wasserstoff, C₁-C₄-Alkyl, OH und O-C₁-C₄-Alkyl,
m, o unabhängig voneinander 0, 1 oder 2 bedeutet und
n 1, 2, 3 oder 4 bedeutet, und
R⁴ Wasserstoff, verzweigtes und unverzweigtes C₁-C₆-Alkyl, Chlor, Brom Fluor, Nitro, Cyano, NR⁴¹R⁴², NH-CO-R⁴³, OR⁴¹, wobei
R⁴¹ und R⁴² unabhängig voneinander Wasserstoff oder C₁-C₄-Alkyl bedeuten und
R⁴³ C₁-C₄-Alkyl oder Phenyl bedeuten, und
R⁵ NR⁵¹R⁵² oder einen der folgenden Reste 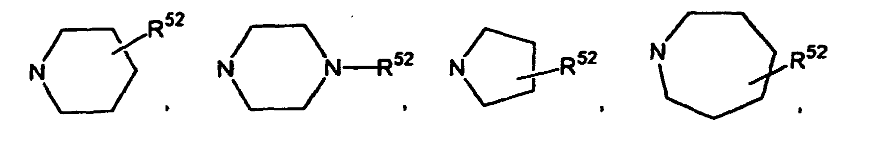 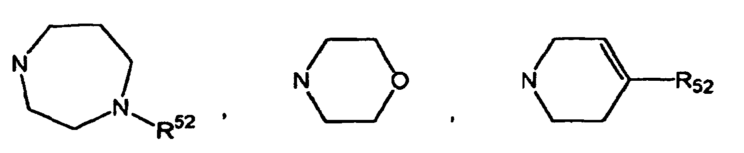
wobei
R⁵¹ Wasserstoff und verzweigtes und unverzweigtes C₁-C₆-Alkyl bedeutet und
R⁵² Wasserstoff, COCH₃, CO-O-C₁-C₄-Alkyl, COCF₃, verzweigtes und unverzweigtes C₁-C₆-Alkyl, wobei ein Wasserstoff des C₁-C₆-Alkylrests durch einen der folgenden Reste substituiert sein kann: OH, O-C₁-C₄-Alkyl und Phenyl und der Phenyl-Ring noch einen oder zwei der folgenden Reste tragen kann: Chlor, Brom, Fluor, verzweigtes und unverzweigtes C₁-C₄-Alkyl, Nitro, Amino, C₁-C₄-Alkylamino, C₁-C₄-Dialkylamino, OH, O-C₁-C₄-Alkyl, CN, SO₂-C₁-C₄-Alkyl, bedeuten kann,
sowie ihre tautomeren Formen, möglichen enantiomeren und diastereomeren Formen, deren Phosphate, Carbamate von Aminosäuren, Ester, sowie mögliche physiologisch verträgliche Salze.

4. Verbindungen nach einem der Ansprüche 1 bis 3, wobei R² in 3-Position und R³ in 4-Position oder R² in 4-Position und R³ in 3-Position zum Benzimidazolring steht.

5. Verbindungen nach einem der Ansprüche 1 bis 4, wobei R¹ und R⁴ Wasserstoff bedeuten.

6. Verbindungen nach einem der Ansprüche 1 bis 5, wobei R² Wasserstoff, verzweigtes oder unverzweigtes C₁-C₆-Alkyl, Nitro, CN, NH₂, O-C₁-C₄-Alkyl bedeutet.

7. Verbindungen nach einem der Ansprüche 1 oder 3 bis 6, wobei
(i) für R³ gleich 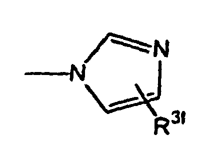
R³¹ Wasserstoff oder -(CH₂)ₚ-R⁵ bedeutet, wobei
p 1 oder 2 bedeutet und
(ii) für R³ gleich 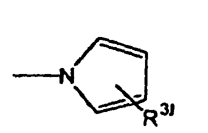
R³¹ Wasserstoff oder -(CH₂)ₚ-R⁵ bedeutet, wobei
p 1 oder 2 bedeutet
und (iii) für R³ gleich 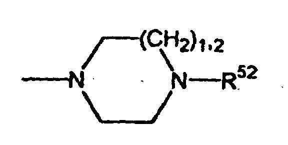
wobei
R⁵² Wasserstoff, verzweigtes und unverzweigtes C₁-C₆-Alkyl bedeutet, wobei ein Wasserstoff des C₁-C₆-Alkyl-rests durch einen der folgenden Reste substituiert sein kann: OH, O-C₁-C₄-Alkyl und Phenyl und der Phenyl-Ring noch einen oder zwei der folgenden Reste tragen kann: Chlor, Brom, Fluor, verzweigtes und unverzweigtes C₁-C₄-Alkyl, Nitro, Amino, C₁-C₄-Alkylamino, C₁-C₄-Dialkylamino, OH, O-C₁-C₄-Alkyl, CN, SO₂-C₁-C₄-Alkyl, bedeuten kann.

8. Verbindungen nach einem der Ansprüche 1, 2 oder 4 bis 6, wobei R³ -O-(CH₂)ₚ-R⁵ mit p gleich 2, 3 oder 4 bedeutet.

9. Verbindungen nach einem der Ansprüche 1, 2 oder 4 bis 7, wobei R⁵ einen 6-gliedrigen Ring und R⁵² einen gegebenenfalls substituierten Phenylring bedeutet.

10. Arzneimittel, enthaltend neben den üblichen Träger und Hilfsstoffen eine Verbindung nach einem der Ansprüche 1 bis 9.

11. Verwendung von Verbindungen der Formel I nach einem der Ansprüche 1 bis 10 zur Herstellung von Arzneimitteln zur Behandlung von Krankheiten, bei denen pathologisch erhöhte Aktivitäten von PARP auftreten.

12. Verwendung von Verbindungen der Formel I nach Anspruch 11 zur Herstellung von Arzneimitteln zur Behandlung von neuro-degenerativen Krankheiten und neuronalen Schädigungen.

13. Verwendung nach Anspruch 11 zur Behandlung von solchen neuro-degenerativen Krankheiten und neuronalen Schädigungen, die durch Ischämie, Trauma oder Massenblutungen ausgelöst werden.

14. Verwendung nach Anspruch 11 zur Behandlung des Schlaganfalls und des Schädel-Himtraumas.

15. Verwendung nach Anspruch 11 zur Behandlung der Alzheimerschen Krankheit der Parkinsonsche Krankheit und der Huntington-Krankheit.

16. Verwendung von Verbindungen der Formel I nach Anspruch 11 zur Herstellung von Arzneimitteln zur Behandlung oder Prophylaxe von Schädigungen durch Ischämien.

17. Verwendung von Verbindungen der Formel I nach Anspruch 11 zur Herstellung von Arzneimitteln zur Behandlung von Epilepsien, insbesondere von generalisierten epileptischen Anfällen, wie zum Beispiel Petit mal und tonischclonische Anfälle und partiell epileptischen Anfällen, wie Temporal Lope, und komplex-partiellen Anfällen.

18. Verwendung von Verbindungen der Formel I nach Anspruch 11 zur Herstellung von Arzneimitteln zur Behandlung von Schädigungen der Nieren nach renalen Ischämien, Schädigungen, die durch medikamentöse Therapie verursacht werden, wie zum Beispiel während der Cyclosporin-Therapie, und zur Behandlung während und nach Nierentransplantationen.

19. Verwendung von Verbindungen der Formel I nach Anspruch 11 zur Herstellung von Arzneimitteln zur Behandlung von Schädigungen des Herzens nach cardialen Ischämien.

20. Verwendung von Verbindungen der Formel I nach Anspruch 11 zur Herstellung von Arzneimitteln zur Behandlung von Mikroinfarkten wie zum Beispiel während und nach Herzklappenersatz, Aneurysmenresektionenen und Herztransplantationen.

21. Verwendung von Verbindungen der Formel I nach Anspruch 11 zur Herstellung von Arzneimitteln zur Behandlung bei einer Revaswlariation kritischer verengter Koronararterien wie zum Beispiel bei PTCA und Bypass-Operationen oder kritisch verengter peripherer Arterien, insbesondere Beinarterien.

22. Verwendung von Verbindungen der Formel I nach Anspruch 11 zur Herstellung von Arzneimitteln zur Behandlung des akuten Myocardinfarktes und von Schädigungen wahrend und nach dessen medikamentöser oder mechanischer Lyse.

23. Verwendung von Verbindungen der Formel I nach Anspruch 11 zur Herstellung von Arzneimitteln zur Behandlung von Tumor en und deren Metastasierung.

24. Verwendung von Verbindungen der Formel I nach Anspruch 11 zur Herstellung von Arzneimitteln zur Behandlung von Sepsis des Multiorganversagens wie zum Beispiel wahrend des septischen Schocks und des "acute respiratory distresssyndroms".

25. Verwendung von Verbindungen der Formel I nach Anspruch 11 zur Herstellung von Arzneimitteln zur Behandlung von immunologischen Krankheiten wie Entzündungen und rheumatische Erkrankungen, wie zum Beispiel rheumatoide Arthritis.

26. Verwendung von Verbindungen der Formel I nach Anspruch 11 zur Herstellung von Arzneimitteln zur Behandlung von Diabetes mellitus.

27. Verbindungen der Formel XX oder XXI 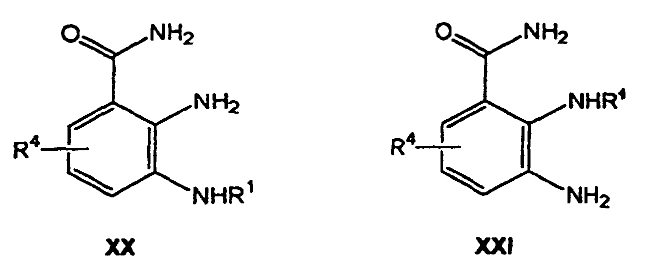 worin
R¹ und R⁴ die in den vorherigen Ansprüchen genannten Bedeutungen haben, sowie ihre Salze.

28. Ein Verfahren zur Herstellung von Verbindungen der Formel XX oder XXI sowie deren Salze, wobei 2-Halogeno-3-nitro-benzoesäureester mit einem geeigneten Diamin in einem polaren Lösungsmittel in Gegenwart einer Base umgesetzt werden und anschließend die Nitrogruppe mit Wasserstoff in Gegenwart eines geeigneten Katalysators hydriert wird.

29. Verwendung von Verbindungen der Formel XX oder XXI in der Synthese von PARP-Inhibitoren.

## Claims

1. Compounds of the formula I or II 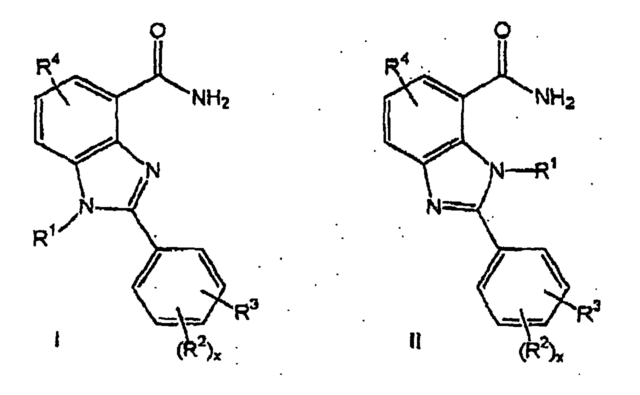 wherein
R¹ denotes hydrogen, branched and unbranched C₁-C₆ alkyl, in which a C atom of the alkyl radical may also carry OR¹¹ or a group R⁵, in which R¹¹ denotes hydrogen or C₁-C₄ alkyl, and
R² denotes hydrogen, chlorine, bromine, iodine, fluorine, CF₃, nitro, NHCOR²¹, NR²²R²³, OH, O-C₁-C₄ alkyl, O-C₁-C₄ alkylphenyl, NH₂, phenyl, in which the phenyl rings may also be substituted with at most two radicals R²⁴, and R²¹ and R²² independently of one another denote hydrogen or C₁-C₄ alkyl and R²³ denotes hydrogen, C₁-C₄ alkyl or phenyl, and R²⁴ denotes OH, C₁-C₆ alkyl, O-C₁-C₄ alkyl, chlorine, bromine, iodine, fluorine, CF₃, nitro, NH₂ and
x may be 0, 1 and 2, and
R³ denotes -D-(F¹)ₚ-(E)_{q}-(F²)ᵣ-G, in which p, q and r may not simultaneously be 0, or denotes -E-(D)ᵤ-(F²)ₛ-(G)ᵥ, in which the radical E may also be substituted with one or two radicals A, and
R⁴ denotes hydrogen, chlorine, fluorine, bromine, iodine, branched and unbranched C₁-C₆ alkyl, OH, nitro, CF₃, CN, NR⁴¹R⁴², NH-CO-R⁴³, O-C₁-C₄ alkyl, in which R⁴¹ and R⁴² independently of one another denote hydrogen or C₁-C₄ alkyl,
and
R⁴³ denotes hydrogen, C₁-C₄ alkylphenyl or phenyl, and
D denotes S and O
E denotes phenyl, imidazole, pyrrole, thiophene, pyridine, pyrimidine, piperazine, pyrazine, furan, thiazole, isoxazole, pyrrolidine, piperidine, trihydroazepine and
F¹ is a chain of 1 to 8 carbon atoms, in which a carbon atom of the chain may also carry an OH or O-C₁-C₄ alkyl group, and
F² is a chain of 1 to 8 carbon atoms, in which a carbon atom of the chain may also carry an OH or O-C₁-C₄ alkyl group, and
p may denote 0 and 1 and
q may be 0 and 1, and
r may be 0 and 1 and
s may be 0 and 1 and
u may be 0 and 1 and
v may be 0 and 1
G may be NR⁵¹R⁵² and 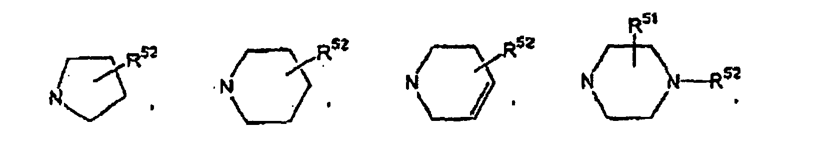 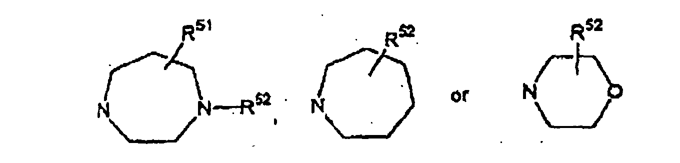 and
R⁵¹ denotes hydrogen and branched and unbranched C₁-C₆ alkyl, (CH₂)ₜ-K, and
R⁵² denotes hydrogen, branched and unbranched C₁-C₆ alkyl, phenyl, 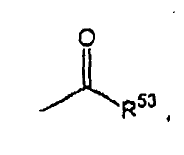 -SO₂R⁵³, -(C=N)R⁵³, -(C=N)NHR⁵³ or -CO-NHR⁵³
wherein
R⁵³ may denote branched or unbranched O-C₁-C₆ alkyl, phenyl, branched or unbranched C₁-C₄ alkylphenyl, in which in R⁵² and R⁵³ independently of one another a hydrogen of the C₁-C₆ alkyl radical may be substituted by one of the following radicals: OH, O-C₁-C₄ alkyl, cyclohexyl, cyclopentyl, tetrahydronaphthyl, cyclopropyl, cyclobutyl, cycloheptyl, naphthyl and phenyl, in which the carbocycles of the radicals R⁵² and R⁵³ independently of one another may also carry one or two of the following radicals: branched or unbranched C₁-C₆ alkyl, branched or unbranched O-C₁-C₄ alkyl, OH, F, Cl, Br, I, CF₃, NO₂, NH₂, CN, COOH, COOC₁-C₄ alkyl, C₁-C₄ alkylamino, CCl₃, C₁-C₄ dialkylamino, SO₂-C₁-C₄ alkyl, SO₂phenyl, CONH₂, CONH-C₁-C₄ alkyl, CONHphenyl, CONH-C₁-C₄ alkylphenyl, NHSO₂-C₁-C₄ alkyl, NHSO₂phenyl, S-C₁-C₄ alkyl, 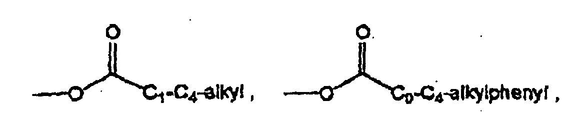 CHO, CH₂-O-C₁-C₄ alkyl, -CH₂O-C₁-C₄ alkylphenyl, -CH₂OH, -SO-C₁-C₄ alkyl, -SO-C₁-C₄ alkylphenyl, -SO₂NH₂, -SO₂NH-C₁-C₄ alkyl,
and two radicals form a bridge -O-(CH₂)_{1,2}-O-, and
A may be hydrogen, chlorine, bromine, iodine, fluorine, CF₃, nitro, OH, O-C₁-C₄ alkyl, O-C₁-C₄ alkylphenyl, NH₂, branched and unbranched C₁-C₆ alkyl, CN, NH-CO-R³³, in which R³³ denotes hydrogen, C₁-C₄ alkyl or phenyl, and
t is 0, 1, 2, 3, 4 and
K denotes phenyl, which may also carry at most two radicals R, NR^{k1}R^{k2} (where R^{k1} and R^{k2} have the same meanings as R⁴¹ and R⁴²), NH-C₁-C₄ alkylphenyl, pyrrolidine, piperidine, 1,2,5,6-tetrahydropyridine, morpholine, trihydroazepine, piperazine, which may also be substituted with an alkyl radical C₁-C₆ alkyl, and homopiperazine, which may also be substituted with an alkyl radical C₁-C₆ alkyl, and
R⁵ may denote hydrogen, C₁-C₆ alkyl, NR⁷R⁹ and 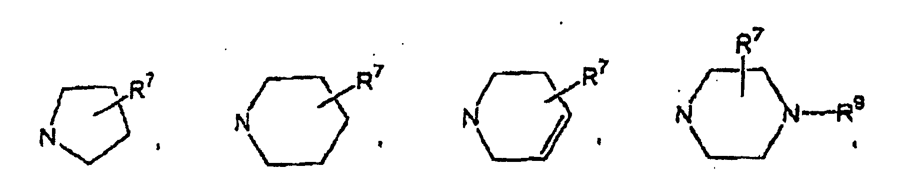 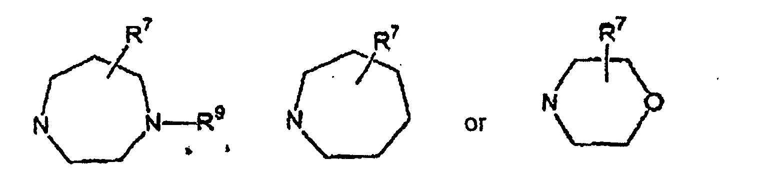
R⁷ denotes hydrogen, C₁-C₆ alkyl, C₁-C₄ alkylphenyl, phenyl, in which the rings may also be substituted with up to two radicals R⁷¹, and
R⁷¹ denotes OH, C₁-C₆ alkyl, O-C₁-C₄ alkyl, chlorine, bromine, iodine, fluorine, CF₃, nitro, NH₂, and
R⁸ denotes hydrogen, C₁-C₆ alkyl, phenyl, C₁-C₄ alkylphenyl, in which the ring may also be substituted with up to two radicals R⁸¹, and
R⁸¹ denotes OH, C₁-C₆ alkyl, O-C₁-C₄ alkyl, chlorine, bromine, iodine, fluorine, CF₃, nitro, NH₂, and
R⁹ denotes hydrogen, COCH₃, CO-O-C₁-C₄ alkyl, COCF₃, branched and unbranched C₁-C₆ alkyl, in which one or two hydrogens of the C₁-C₆ alkyl radical may be substituted in each case by one of the following radicals: OH, O-C₁-C₄ alkyl and phenyl and the phenyl ring may also carry one or two of the following radicals: iodine, chlorine, bromine, fluorine, branched and unbranched C₁-C₆ alkyl, nitro, amino, C₁-C₄ alkylamino, C₁-C₄ dialkylamino, OH, O-C₁-C₄ alkyl, CN, CF₃, SO₂-C₁-C₄ alkyl,
as well as their tautomeric forms, possible enantiomeric and diastereomeric forms, and their phosphates, carbamates of amino acids, esters and pharmacologically compatible salts.

2. Compounds of the general formula I or II according to claim 1, wherein
R¹ denotes hydrogen, branched and unbranched C₁-C₆ alkyl, in which a C atom of the alkyl radical may also carry OR¹¹ or a group R⁵, in which
R¹¹ denotes hydrogen or C₁-C₄ alkyl, and
R² denotes hydrogen, chlorine, fluorine, bromine, iodine, branched and unbranched C₁-C₆ alkyl, nitro, CF₃, CN, NR²¹R²², NH-CO-R²³, OR²¹, in which
R²¹ and R²¹ independently of one another denote hydrogen or C₁-C₄ alkyl and
R²³ denotes hydrogen, C₁-C₄ alkyl or phenyl, and
R³ denotes -O-(CH₂)ₒ-(CHR³¹)ₘ-(CH₂)ₙ-R⁵, in which
R³¹ denotes hydrogen, C₁-C₄ alkyl, OH and O-C₁-C₄ alkyl,
m, o independently of one another denotes 0, 1 or 2, and
n denotes 1, 2, 3 or 4, and
R⁴ denotes hydrogen, branched and unbranched C₁-C₆ alkyl, chlorine, bromine, fluorine, nitro, cyano, NR⁴¹R⁴², NH-CO-R⁴³, OR⁴¹, in which
R⁴¹ and R⁴² independently of one another denote hydrogen or C₁-C₄ alkyl, and
R⁴³ denotes C₁-C₄ alkyl or phenyl, and
R⁵ denotes NR⁵¹R⁵³ or one of the following radicals 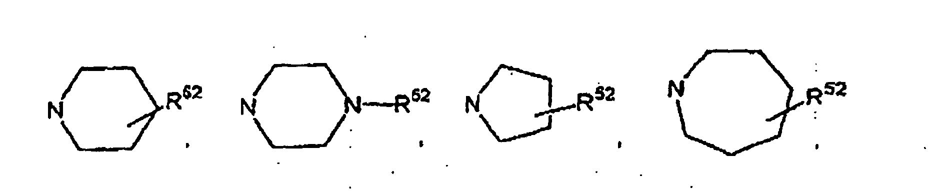 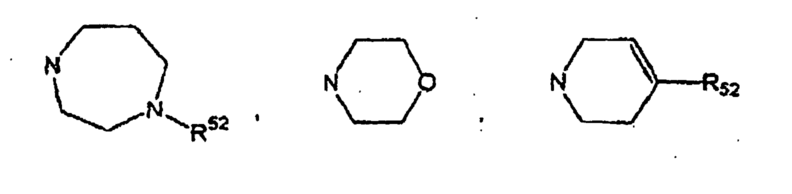
in which
R⁵¹ denotes hydrogen and branched and unbranched C₁-C₆ alkyl, and
R⁵² denotes hydrogen, branched and unbranched C₁-C₆ alkyl, phenyl, 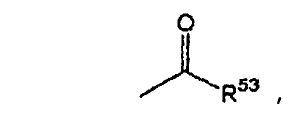 -SO₂R⁵³, wherein
R⁵³ may denote branched and unbranched O-C₁-C₆ alkyl, phenyl, branched or unbranched C₁-C₄ alkylphenyl, in which in R⁵² and R⁵³ independently of one another a hydrogen of the C₁-C₆ alkyl radical may be substituted by one of the following radicals: OH, O-C₁-C₄ alkyl, cyclohexyl, cyclopentyl, tetrahydronaphthyl, cyclopropyl, cyclobutyl, cycloheptyl, naphthyl and phenyl, in which the carbocycles of the radicals R⁵² and R⁵³ independently of one another may also carry one or two of the following radicals: branched or unbranched C₁-C₆ alkyl, branched or unbranched O-C₁-C₄ alkyl, OH, F, Cl, Br, I, CF₃, NO₂, NH₂, CN, COOH, COOC₁-C₄ alkyl, C₁-C₄ alkylamino, CCl₃, C₁-C₄ dialkylamino, SO₂-C₁-C₄ alkyl, SO₂phenyl, CONH₂, CONH-C₁-C₄ alkyl, CONHphenyl, CONH-C₁-C₄ alkylphenyl, NHSO₂-C₁-C₄ alkyl, NHSO₂phenyl, S-C₁-C₄ alkyl, 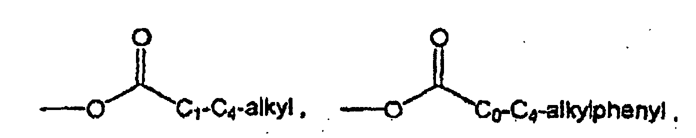 CHO, CH₂-O-C₁-C₄ alkyl, -CH₂O-C₁-C₄ alkylphenyl, -CH₂OH, -SO-C₁-C₄ alkyl, -SO-C₁-C₄ alkylphenyl, -SO₂-NH₂, -SO₂-NH-C₁-C₄ alkyl, and two radicals form a bridge -O-(CH₂)_{1,2}-O-,
as well as their tautomeric forms, possible enantiomeric and diastereomeric forms, their phosphates, carbamates of amino acids, esters as well as possible pharmacologically compatible salts.

3. Compounds of the general formula I or II according to claim 1, wherein
R¹ denotes hydrogen, branched and unbranched C₁-C₆ alkyl, in which a C atom of the alkyl radical may also carry OR¹¹ or a group R⁵, in which
R¹¹ denotes hydrogen or C₁-C₄ alkyl, and
R² denotes hydrogen, chlorine, fluorine, bromine, iodine, branched and unbranched C₁-C₆ alkyl, nitro, CF₃, CN, NR²¹R²², NH-CO-R²³, OR²¹, in which
R²¹ and R²² independently of one another denote hydrogen or C₁-C₄ alkyl and
R²³ denotes hydrogen, C₁-C₄ alkyl or phenyl, and
R³ denotes 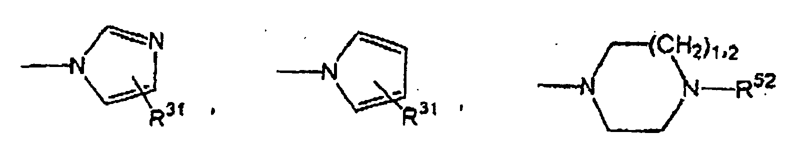 and
R³¹ denotes hydrogen, CHO and -(CH₂)ₒ-(CHR³²)ₘ-(CH₂)ₙ-R⁵, in which R³² denotes hydrogen, C₁-C₄ alkyl, OH and O-C₁-C₄ alkyl,
m, o independently of one another denotes 0, 1 or 2, and
n denotes 1, 2, 3 or 4, and
R⁴ denotes hydrogen, branched and unbranched C₁-C₆ alkyl, chlorine, bromine, fluorine, nitro, cyano, NR⁴¹R⁴², NH-CO-R⁴³, OR⁴¹, in which
R⁴¹ and R⁴² independently of one another denote hydrogen or C₁-C₄ alkyl, and
R⁴³ denotes C₁-C₄ alkyl or phenyl, and
R⁵ denotes NR⁵¹R⁵³ or one of the following radicals 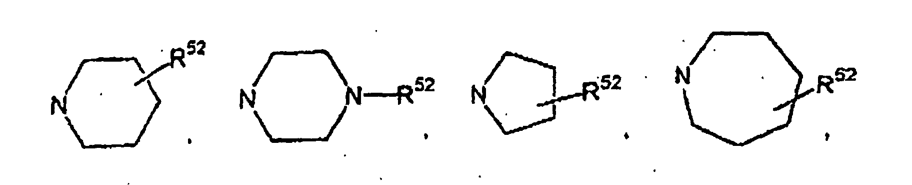 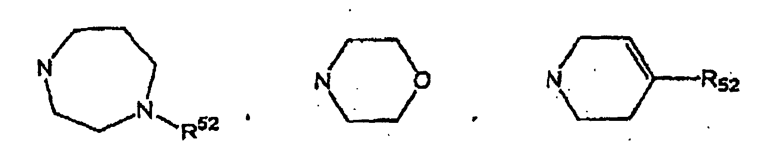
in which
R⁵¹ denotes hydrogen and branched and unbranched C₁-C₆ alkyl, and
R⁵² denotes hydrogen, COCH₃, CO-O-C₁-C₄ alkyl, COCF₃, branched and unbranched C₁-C₆ alkyl, in which a hydrogen of the C₁-C₆ alkyl radical may be substituted by one of the following radicals: OH, O-C₁-C₄ alkyl and phenyl and the phenyl ring may also carry one or two of the following radicals: chlorine, bromine, fluorine, branched and unbranched C₁-C₄ alkyl, nitro, amino, C₁-C₄ alkylamino, C₁-C₄ dialkylamino, OH, O-C₁-C₄ alkyl, CN, SO₂-C₁-C₄ alkyl,
as well as their tautomeric forms, possible enantiomeric and diastereomeric forms, their phosphates, carbamates of amino acids, esters, as well as possible pharmacologically compatible salts.

4. Compounds according to one of claims 1 to 3, in which R² is in the 3-position and R³ is in the 4-position or R² is in the 4-position and R³ is in the 3-position to the benzimidazole ring.

5. Compounds according to one of claims 1 to 4, in which R¹ and R⁴ denote hydrogen.

6. Compounds according to one of claims 1 to 5, in which R² denotes hydrogen, branched or unbranched C₁-C₆ alkyl, nitro, CN, NH₂, O-C₁-C₆ alkyl.

7. Compounds according to one of claims 1 or 3 to 6, in which
(i) for R³ identical to 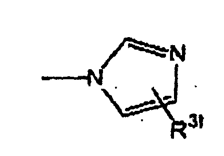
R³¹ denotes hydrogen or -(CH₂)ₚ-R⁵, in which
p denotes 1 or 2, and
(ii) for R³ identical to 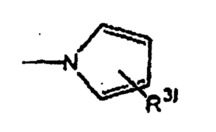
R³¹ denotes hydrogen or -(CH₂)ₚ-R⁵, in which
p denotes 1 or 2,
and (iii) for R³ identical to 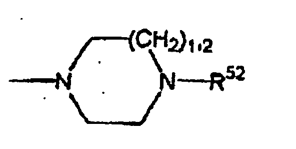
wherein
R⁵² denotes hydrogen, branched and unbranched C₁-C₆ alkyl, in which a hydrogen of the C₁-C₆ alkyl radical may be substituted by one of the following radicals: OH, O-C₁-C₄ alkyl and phenyl and the phenyl ring may also carry one or two of the following radicals: chlorine, bromine, fluorine, branched and unbranched C₁-C₄ alkyl, nitro, amino, C₁-C₄ alkylamino, C₁-C₄ dialkylamino, OH, O-C₁-C₄ alkyl, CN, SO₂-C₁-C₄ alkyl.

8. Compounds according to one of claims 1, 2 or 4 to 6, in which R³ denotes -O-(CH₂)ₚ-R⁵ where p is equal to 2, 3 or 4.

9. Compounds according to one of claims 1, 2 or 4 to 7, in which R⁵ denotes a 6-membered ring and R⁵² denotes an optionally substituted phenyl ring.

10. Medicament containing a compound according to one of claims 1 to 9 in addition to the conventional carriers and auxiliary substances.

11. Use of compounds of the formula I according to one of claims 1 to 10 for the production of medicaments for treating diseases in which pathologically raised activities of PARP occur.

12. Use of compounds of the formula I according to claim 11 for the production of medicaments for treating neurodegenerative diseases and neuronal damage.

13. Use according to claim 11 for the treatment of such neurodegenerative diseases and neuronal damage that are triggered by ischaemia, trauma or massive bleeding.

14. Use according to claim 11 for the treatment of strokes and craniocerebral trauma.

15. Use according to claim 11 for the treatment of Alzheimer's disease, Parkinson's disease and Huntington's disease.

16. Use of compounds of the formula I according to claim 11 for the production of medicaments for the treatment or prophylaxis of damage due to ischaemia.

17. Use of compounds of the formula I according to claim 11 for the production of medicaments for treating epilepsy, in particular generalised epileptic attacks, such as for example petit mal and tonic-clonic attacks and partial epileptic attacks such as temporal lobe and complex-partial attacks.

18. Use of compounds of the formula I according to claim 11 for the production of medicaments for treating kidney damage after renal ischaemia, damage caused by medical treatment, such as for example during cyclosporin therapy, and for treatment during and after kidney transplants.

19. Use of compounds of the formula I according to claim 11 for the production of medicaments for treating cardiac damage after cardiac ischaemia.

20. Use of compounds of the formula I according to claim 11 for the production of medicaments for treating microinfarcts, such as for example during and after heart valve replacement, aneurysm resections and heart transplants.

21. Use of compounds of the formula I according to claim 11 for the production of medicaments for treatment involving a revascularisation of critically constricted coronary arteries, such as for example in PTCA and bypass operations, or critically constricted peripheral arteries, in particular leg arteries.

22. Use of compounds of the formula I according to claim 11 for the production of medicaments for the treatment of acute myocardial infarction and damage during and after its medical or mechanical lysis.

23. Use of compounds of the formula I according to claim 11 for the production of medicaments for treating tumours and metastases.

24. Use of compounds of the formula I according to claim 11 for the production of medicaments for treating sepsis in multi-organ failure, such as for example during septic shock and acute respiratory distress syndrome.

25. Use of compounds of the formula I according to claim 11 for the production of medicaments for treating immunological diseases such as inflammatory and rheumatic diseases, such as for example rheumatoid arthritis.

26. Use of compounds of the formula I according to claim 11 for the production of medicaments for treating diabetes mellitus.

27. Compounds of the formula XX or XXI 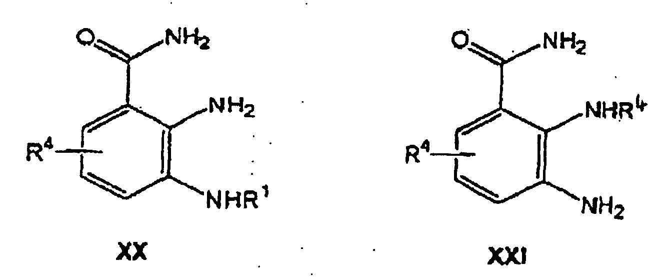 wherein
R¹ and R⁴ have the meanings given in the preceding claims, as well as their salts.

28. A process for the production of compounds of the formula XX or XXI as well as their salts, in which 2-halogeno-3-nitrobenzoic acid esters are reacted with a suitable diamine in a polar solvent in the presence of a base and the nitro group is then hydrogenated with hydrogen in the presence of a suitable catalyst.

29. Use of compounds of the formula XX or XXI in the synthesis of PARP inhibitors.

## Revendications

1. Composés de formule I ou II 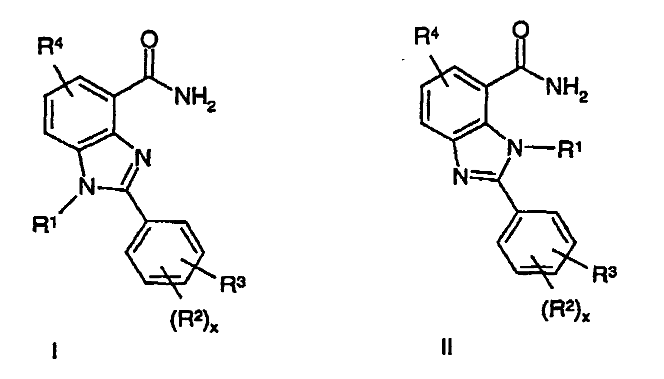 où
R¹ représente l'hydrogène, C₁-C₆-alkyle ramifié et non ramifié, où un atome de carbone du groupement alkyle peut porter encore OR¹¹ ou un groupe R⁵, où R¹¹ représente l'hydrogène où C₁-C₄-alkyle, et
R² représente l'hydrogène, le chlore, le brome, l'iode, le fluor, CF₃, nitro, NHCOR²¹, NR²²R²³, OH, O-C₁-C₄-alkyle, O-C₁-C₄-alkyl-phényle, NH₂, phényle, où les cycles phényle peuvent être substitués encore avec au maximum deux groupements R²⁴, et R²¹ et R²² représentent indépendamment l'un de l'autre l'hydrogène ou C₁-C₄-alkyle et R²³ représente l'hydrogène, C₁-C₄-alkyle ou phényle, et R²⁴ représente OH, C₁-C₆-alkyle, O-C₁-C₄-alkyle, le chlore, le brome, l'iode, le fluor, CF₃, nitro, NH₂ et
x peut être 0, 1 ou 2 et
R³ représente -D-(F¹)ₚ-(E)_{q}-(F²)ᵣ-G, où p, q et r ne peuvent pas être simultanément 0, ou -E-(D)ᵤ-(F²)ₛ-(G)ᵥ, où le groupement E peut être substitué encore avec un ou deux groupements A, et
R⁴ représente l'hydrogène, le chlore, le fluor, le brome, l'iode, C₁-C₆-alkyle ramifié et non ramifié, OH, nitro, CF₃, CN, NR⁴¹R⁴², NH-CO-R⁴³, O-C₁-C₄-alkyle, où R⁴¹ et R⁴² représentent indépendamment l'un de l'autre l'hydrogène ou C₁-C₄-alkyle et
R⁴³ représente l'hydrogène, C₁-C₄-alkyle, C₁-C₄-alkyl-phényle ou phényle, et
D représente S et O
E représente phényle, imidazole, pyrrole, thiophène, pyridine, pyrimidine, pipérazine, pyrazine, furane, thiazole, isoxazole, pyrrolidine, pipéridine, trihydroazépine et
F¹ représente une chaîne de 1 à 8 atomes de carbone où un atome de carbone de la chaîne peut porter encore un groupe OH ou O-C₁-C₄-alkyle et
F² représente une chaîne de 1 à 8 atomes de carbone, où un atome de carbone de la chaîne peut porter encore un groupe OH ou O-C₁-C₄-alkyle et
p peut représenter 0 et 1 et
q peut être 0 et 1, et
r peut être 0 et 1 et
s peut être 0 et 1 et
u peut être 0 et 1 et
v peut être 0 et 1
G peut être NR⁵¹R⁵² et 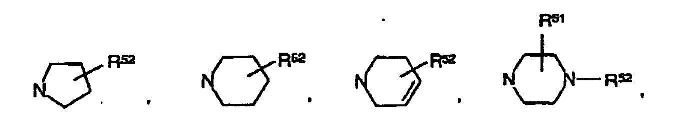 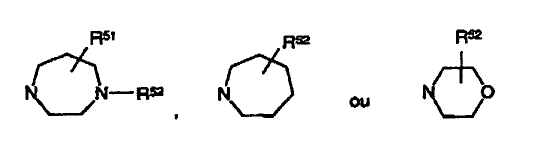 et
R⁵¹ représente l'hydrogène et C₁-C₆-alkyle ramifié et non ramifié, (CH₂)ₜ-K et
R⁵² représente l'hydrogène, C₁-C₆-alkyle ramifié et non ramifié, phényle, 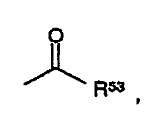 -SO₂R⁵³, -(C=N)R⁵³, -(C=N)-NHR⁵³ ou -CO-NHR⁵³
où
R⁵³ peut représenter O-C₁-C₆-alkyle ramifié ou non ramifié, phényle, C₁-C₄-alkyl-phényle ramifié ou non ramifié, où, dans R⁵² et R⁵³, indépendamment l'un de l'autre un hydrogène du groupement C₁-C₆-alkyle peut être substitué par l'un des groupements suivants : OH, O-C₁-C₄-alkyle, cyclohexyle, cyclopentyle, tétrahydronaphtyle, cyclopropyle, cyclobutyle, cycloheptyle, naphtyle et phényle, où les carbocycles des groupements R⁵² et R⁵³ peuvent porter indépendamment l'un de l'autre encore un ou deux des groupements suivants : C₁-C₆-alkyle ramifié ou non ramifié, O-C₁-C₄-alkyle ramifié ou non ramifié, OH, F, Cl, Br, I, CF₃, NO₂, NH₂, CN, COOH, COOC₁-C₄-alkyle, C₁-C₄-alkyl-amino, CCl₃, C₁-C₄-dialkylamino, SO₂-C₁-C₄-alkyle, SO₂phényle, CONH₂, CONH-C₁-C₄-alkyle, CONHphényle, CONH-C₁-C₄-alkyl-phényle, NHSO₂-C₁-C₄-alkyle, NHSO₂phényle, S-C₁-C₄-alkyle, 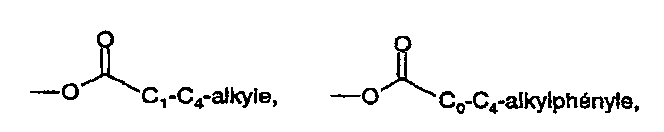 CHO, CH₂-O-C₁-C₄-alkyle, -CH₂O-C₁-C₄-alkyl-phényle, -CH₂OH, -SO-C₁-C₄-alkyle, -SO-C₁-C₄-alkyl-phényle, -SO₂NH₂, -SO₂NH-C₁-C₄-alkyle
et deux groupements forment un pont -O-(CH₂)_{1,2}-O-,
A peut être l'hydrogène, le chlore, le brome, l'iode, le fluor, CF₃, nitro, OH, O-C₁-C₄-alkyle, O-C₁-C₄-alkyl-phényle, NH₂, C₁-C₆-alkyle ramifié et non ramifié, CN, NH-CD-R³³, où R³³ représente l'hydrogène, C₁-C₄-alkyle ou phényle
et
t représente 0, 1, 2, 3, 4 et
K représente phényle, qui peut porter encore au maximum deux groupements R, NR^{k1}R^{k2} (R^{k1} et R^{k2} ayant les mêmes significations que R⁴¹ et R⁴²), NH-C₁-C₄-alkyl-phényle, pyrrolidine, pipéridine, 1,2,5,6-tétrahydropyridine, morpholine, trihydroazépine, pipérazine, qui peut encore être substitué par un groupement alkyle C₁-C₆-alkyle, et homopipérazine, qui peut encore être substitué par un groupement alkyle C₁-C₆-alkyle, et
R⁵ peut représenter l'hydrogène, C₁-C₆-alkyle, NR⁷R⁹ et 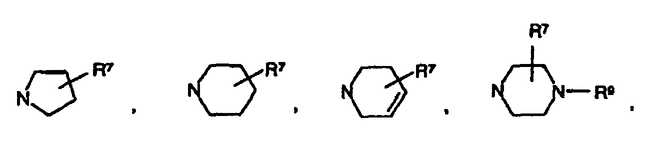 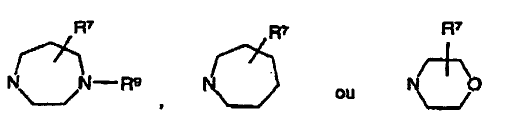 et
R⁷ peut représenter l'hydrogène, C₁-C₆-alkyle, C₁-C₄-alkyl-phényle, phényle,
où les cycles peuvent être substitués encore par jusqu'à deux groupements R⁷¹,
et
R⁷¹ peut représenter OH, C₁-C₆-alkyle, O-C₁-C₄-alkyle, le chlore, le brome, l'iode, le fluor, CF₃, nitro, NH₂,
R⁸ peut représenter l'hydrogène, C₁-C₆-alkyle, phényle, C₁-C₄-alkyl-phényle, où le cycle peut être substitué encore par jusqu'à deux groupements R⁸¹, et
R⁸¹ peut représenter OH, C₁-C₆-alkyle, O-C₁-C₄-alkyle, le chlore, le brome, l'iode, le fluor, CF₃, nitro, NH₂, et
R⁹ peut représenter l'hydrogène, COCH₃, CO-O-C₁-C₄-alkyle, COCF₃, C₁-C₆-alkyle ramifié et non ramifié, où un ou deux hydrogènes du groupement C₁-C₆-alkyle peuvent être substitués dans chaque cas par l'un des groupements suivants: OH, O-C₁-C₄-alkyle et phényle et le cycle phényle peut porter encore un ou deux des groupements suivants : iode, chlore, brome, fluor, C₁-C₆-alkyle ramifié et non ramifié, nitro, amino, C₁-C₄-alkylamino, C₁-C₄-dialkylamino, OH, O-C₁-C₄-alkyle, CN, CF₃, SO₂-C₁-C₄-alkyle,
ainsi que leurs formes tautomères, leurs formes énantiomères et diastéréoisomères possibles et leurs phosphates, carbamates d'aminoacides, esters et sels pharmacologiquement acceptables.

2. Composés de formule générale I ou II selon la revendication 1 où
R¹ peut représenter l'hydrogène, C₁-C₆-alkyle ramifié et non ramifié, où un atome de carbone du groupement alkyle peut porter encore OR¹¹ ou un groupe R⁵ où
R¹¹ représente l'hydrogène ou C₁-C₄-alkyle, et
R² représente l'hydrogène, le chlore, le fluor, le brome, l'iode, C₁-C₆-alkyle ramifié et non ramifié, nitro, CF₃, CN, NR²¹R²², NH-CO-R²³, OR²¹ où
R²¹ et R²² représentent Indépendamment l'un de l'autre l'hydrogène ou C₁-C₄-alkyle et
R²³ représente l'hydrogène, C₁-C₄-alkyle ou phényle, et
R³ représente -O-(CH₂)ₒ-(CHR³¹)ₘ-(CH₂)ₙ-R⁵, où
R³¹ représente l'hydrogène, C₁-C₄-alkyle, OH et O-C₁-C₄-alkyle,
m, o représentent indépendamment l'un de l'autre 0, 1 ou 2, et
n représente 1, 2, 3 ou 4, et
R⁴ représente l'hydrogène, C₁-C₆-alkyle ramifié et non ramifié, le chlore, le brome, le fluor, nitro, cyano, NR⁴¹R⁴², NH-CO-R⁴³, OR⁴¹, où
R⁴¹ et R⁴² représentent Indépendamment l'un de l'autre l'hydrogène ou C₁-C₄-alkyle et
R⁴³ représente C₁-C₄-alkyle ou phényle, et
R⁵ représente NR⁵¹R⁵² ou l'un des groupements suivants 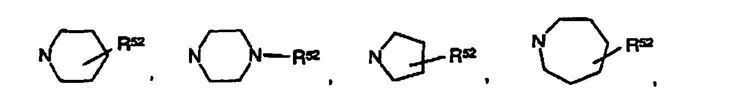 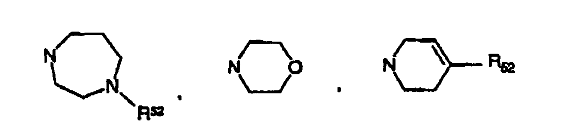
où
R⁵¹ représente l'hydrogène et C₁-C₆-alkyle ramifié et non ramifié et
R⁵² représente l'hydrogène, C₁-C₆-alkyle ramifié et non ramifié, phényle, 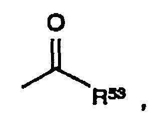 -SO₂R⁵³, où
R⁵³ représente O-C₁-C₆-alkyle ramifié ou non ramifié, phényle, C₁-C₄-alkyl-phényle ramifié ou non ramifié, où, dans R⁵² et R⁵³, indépendamment l'un de l'autre un hydrogène du groupement C₁-C₆-alkyle peut être substitué par l'un des groupements suivants : OH, O-C₁-C₄-alkyle, cyclohexyle, cyclopentyle, tétrahydronaphtyle, cyclopropyle, cyclobutyle, cycloheptyle, naphtyle et phényle, où les carbocydes des groupements R⁵² et R⁵³ peuvent porter encore indépendamment l'un de l'autre un ou deux des groupements suivants : C₁-C₆-alkyle ramifié ou non ramifié, O-C₁-C₄-alkyle ramifié ou non ramifié, OH, F, Cl, Br, I, CF₃, NO₂, NH₂, CN, COOH, COOC₁-C₄-alkyle, C₁-C₄-alkyl-amino, CCl₃, C₁-C₄-dialkylamino, SO₂-C₁-C₄-alkyle, SO₂phényle, CONH₂, CONH-C₁-C₄-alkyle, CONHphényle, CONH-C₁-C₄-alkyl-phényle, NHSO₂-C₁-C₄-alkyle, NHSO₂phényle, S-C₁-C₄-alkyle, 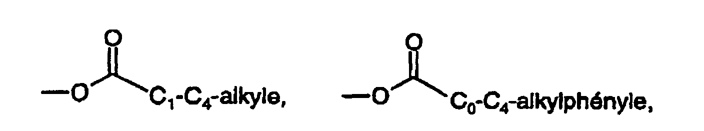 CHO, CH₂-O-C₁-C₄-alkyle, -CH₂O-C₁-C₄-alkyl-phényle, -CH₂OH, -SO-C₁-C₄-alkyle, -SO-C₁-C₄-alkyl-phényle, -SO₂NH₂, -SO₂NH-C₁-C₄-alkyle
et deux groupements forment un pont -O-(CH₂)_{1,2}-O-,
ainsi que leurs formes tautomères, leurs formes énantiomères et diastéréoisomères possibles, leurs phosphates, carbamates d'aminoacides, esters ainsi que leurs sels physiologiquement acceptables.

3. Composés de formule générale I ou II selon la revendication 1, où
R¹ représente l'hydrogène, C₁-C₆-alkyle ramifié et non ramifié, où un atome de carbone du groupement alkyle peut porter encore OR¹¹ ou un groupe R⁵, où
R¹¹ représente l'hydrogène ou C₁-C₄-alkyle, et
R² représente l'hydrogène, le chlore, le fluor, le brome, l'iode, C₁-C₆-alkyle ramifié et non ramifié, nitro, CF₃, CN, NR²¹R²², NH-CO-R²³, OR²¹, où
R²¹ et R²² représentent Indépendamment l'un de l'autre l'hydrogène ou C₁-C₄-alkyle et
R²³ représente l'hydrogène, C₁-C₄-alkyle ou phényle, et
R³ représente 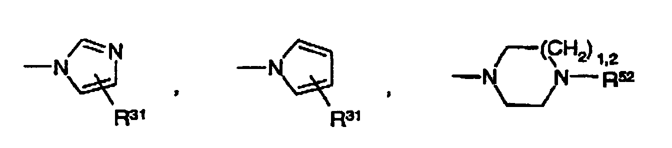 et
R³¹ représente l'hydrogène, CHO et -(CH₂)ₒ-(CHR³²)ₘ-(CH₂)ₙ-R⁵ où R³² représente l'hydrogène, C₁-C₄-alkyle, OH et O-C₁-C₄-alkyle,
m, o représentent indépendamment l'un de l'autre 0, 1 ou 2 et
n représente 1, 2, 3 ou 4, et
R⁴ représente l'hydrogène, C₁-C₆-alkyle ramifié et non ramifié, le chlore, le brome, le fluor, nitro, cyano, NR⁴¹R⁴², NH-CO-R⁴³, OR⁴¹, où
R⁴¹ et R⁴² représentent indépendamment l'un de l'autre l'hydrogène ou C₁-C₄-alkyle et
R⁴³ représente C₁-C₄-alkyle ou phényle, et
R⁵ représente NR⁵¹R⁵² ou l'un des groupements suivants 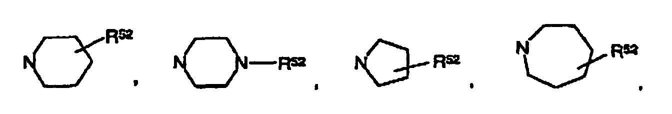 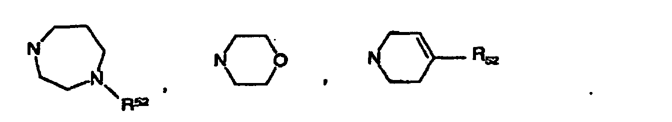
où
R⁵¹ représente l'hydrogène et C₁-C₆-alkyle ramifié et non ramifié et
R⁵² peut représenter l'hydrogène, COCH₃, CO-O-C₁-C₄-alkyle, COCF₃, C₁-C₆-alkyle ramifié et non ramifié, où un hydrogène du groupement C₁-C₆-alkyle peut être substitué par l'un des groupements suivants : OH, O-C₁-C₄-alkyle et phényle et le cycle phényle peut porter encore un ou deux des groupements suivants : chlore, brome, fluor, C₁-C₄-alkyle ramifié et non ramifié, nitro, amino, C₁-C₄-alkylamino, C₁-C₄-dialkylamino, OH, O-C₁-C₄-alkyle, CN, SO₂-C₁-C₄-alkyle,
ainsi que leurs formes tautomères, leurs fermes énantiomères et diastéréoisomères possibles, leurs phosphates, carbamates d'aminoacides, esters, ainsi que leurs sels physiologiquement acceptables possibles.

4. Composés selon l'une des revendications 1 à 3 où R² est en position 3 et R³ est en position 4 ou R² est en position 4 et R³ est en position 3 du cycle benzimidazole.

5. Composés selon l'une des revendications 1 à 4 où R¹ et R⁴ représentent l'hydrogène.

6. Composés selon l'une des revendications 1 à 5 où R² représente l'hydrogène, C₁-C₆-alkyle ramifié ou non ramifié, nitro, CN, NH₂, O-C₁-C₄-alkyle.

7. Composés selon l'une des revendications 1 ou 3 à 6, où
(i) pour R³ égal à 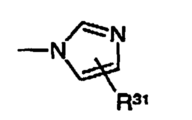
R³¹ représente l'hydrogène ou -(CH₂)ₚ-R⁵, où
p représente 1 ou 2 et
(ii) pour R³ égal à 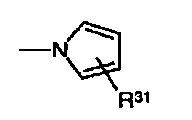
R³¹ représente l'hydrogène ou -(CH₂)ₚ-R⁵, où
p représente 1 ou 2
et (iii) pour R³ égal à 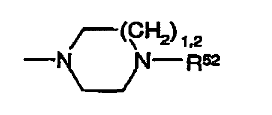
où
R⁵² représente l'hydrogène, C₁-C₆-alkyle ramifié et non ramifié, où un hydrogène du groupement C₁-C₆-alkyle peut être substitué par l'un des groupements suivants : OH, O-C₁-C₄-alkyle et phényle et le cycle phényle peut porter encore un ou deux des groupements suivants : chlore, brome, fluor, C₁-C₄-alkyle ramifié et non ramifié, nitro, amino, C₁-C₄-alkylamino, C₁-C₄-dialkylamino, OH, O-C₁-C₄-alkyle, CN, SO₂-C₁-C₄-alkyle.

8. Composés selon l'une des revendications 1, 2 ou 4 à 6 où R³ représente -O-(CH₂)ₚ-R⁵ avec p égal à 2, 3 ou 4.

9. Composés selon l'une des revendications 1, 2 ou 4 à 7, où R⁵ représente un cycle à six chaînons et R⁵² représente un cycle phényle éventuellement substitué.

10. Médicament contenant un composé selon l'une des revendications 1 à 9 outre les supports et adjuvants courants.

11. Utilisation de composés de formule I selon l'une des revendications 1 à 10 pour la production de médicaments pour le traitement de maladies dans lesquelles des activités pathologiquement augmentées de PARP apparaissent.

12. Utilisation de composés de formule I selon la revendication 11 pour la production de médicaments pour le traitement de maladies neurodégénératives et de lésions neuronales.

13. Utilisation selon la revendication 11 pour le traitement de maladies neurodégénératives et de lésions neuronales qui sont déclenchées par une ischémie, un traumatisme ou des hémorragies massives.

14. Utilisation selon la revendication 11 pour le traitement de l'attaque d'apoplexie et du traumatisme crânien-cérébral.

15. Utilisation selon ta revendication 11 pour le traitement de la maladie d'Alzheimer, de la maladie de Parkinson et de la maladie de Huntington.

16. Utilisation de composés de formule I selon la revendication 11 pour la production de médicaments pour le traitement ou la prophylaxie de lésions dues à des ischémies.

17. Utilisation de composés de formule I selon la revendication 11 pour la production de médicaments pour le traitement d'épilepsies, en particuller de crises épileptiques généralisées, comme par exemple le petit mal et de crises tonico-doniques et de crises partiellement épileptiques, comme Temporal Lope, et de crises partielles complexes.

18. Utilisation de composés de formule I selon la revendication 11 pour la production de médicaments pour le traitement de lésions des reins après des ischémies rénales, de lésions qui sont provoquées par une thérapie médicamenteuse, comme par exemple pendant la thérapie à la cyclosporine, et pour le traitement pendant et après des transplantations rénales.

19. Utilisation de composés de formule I selon la revendication 11 pour la production de médicaments pour le traitement de lésions du coeur après des ischémies cardiaques.

20. Utilisation de composés de formule I selon la revendication 11 pour la production de médicaments pour le traitement de micro-Infarctus comme par exemple pendant et après un remplacement de valves cardiaques, des résections d'anévrismes et des transplantations cardiaques.

21. Utilisation de composés de formule I selon la revendication 11 pour la production de médicaments pour le traitement d'artères coronaires rétrécies de manière critique lors d'une revascularisation, comme par exemple au cours de PTCA et d'opérations de pontage, ou d'artères périphériques rétrécies de manière critique, en particulier d'artères des jambes.

22. Utilisation de composés de formule I selon la revendication 11 pour la production de médicaments pour le traitement de l'infarctus du myocarde aigu et de lésions pendant et après sa lyse médicamenteuse ou mécanique.

23. Utilisation de composés de formule I selon la revendication 11 pour la production de médicaments pour le traitement de tumeurs et de leurs métastases.

24. Utilisation de composés de formule I selon la revendication 11 pour la production de médicaments pour le traitement de la septicémie de la défaillance d'organes multiples comme par exemple pendant le choc septique et le "acute respiratory distress-syndrom".

25. Utilisation de composés de formule I selon la revendication 11 pour la production de médicaments pour le traitement de maladies immunologiques comme les inflammations et de maladies rhumatismales comme par exemple la polyarthrite rhumatoïde.

26. Utilisation de composés de formule I selon la revendication 11 pour la production de médicaments pour le traitement du diabète sucré.

27. Composés de formule XX ou XXI 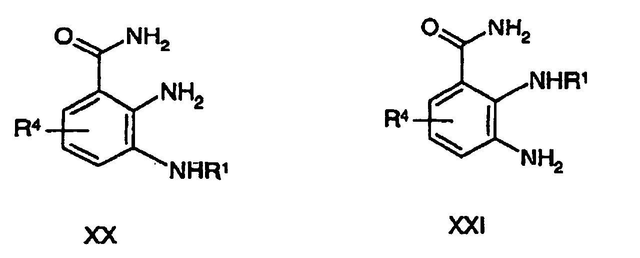 où
R¹ et R⁴ ont les significations citées dans les revendications précédentes, ainsi que leurs sels.

28. Procédé de production de composés de formule XX ou XXI ainsi que de leurs sels, où des esters d'adde 2-halogéno-3-nitrobenzoïque sont mis à réagir avec une diamine appropriée dans un solvant polaire en présence d'une base puis le groupe nitro est hydrogéné avec l'hydrogène en présence d'un catalyseur approprié.

29. Utilisation de composés de formule XX ou XXI dans la synthèse d'inhibiteurs de PARP.
